(19) Europäisches Patentamt
European Patent Office
Office européen des brevets

(11) Publication number: **0 005 889**
**B1**

(12) # EUROPEAN PATENT SPECIFICATION

(45) Date of publication of patent specification: **16.06.82**

(21) Application number: **79200271.9**

(22) Date of filing: **01.06.79**

(51) Int. Cl.[3]: **C 07 D 205/08,**
C 07 D 499/00,
C 07 D 501/00,
C 07 D 501/04,
A 61 K 31/43,
A 61 K 31/545

(54) **Process for the preparation of beta-lactam derivatives, the novel derivatives so obtainable and pharmaceutical compositions containing them.**

(30) Priority: **02.06.78 US 911858**

(43) Date of publication of application:
**12.12.79 Bulletin 79/25**

(45) Publication of the grant of the patent:
**16.06.82 Bulletin 82/24**

(84) Designated Contracting States:
**AT BE CH DE FR GB IT NL SE**

(56) References cited:
**DE - A - 2 751 624**
**DE - A - 2 811 514**
**DE - A - 2 822 001**
**US - A - 4 076 826**
**US - A - 4 078 068**
**US - A - 4 123 539**

**E. H. Flynn —Cephalosporins and Penicillins. Academic Press (1972). 577—578.**

**IUPAC Rules for the nomenclature of organic chemistry (1979) et A 13.5.**

(73) Proprietor: **SCHERICO LTD.**
**Töpferstrasse 5**
**CH-6004 Lucerne (CH)**

(72) Inventor: **Ganguly, Ashit Kumar**
**96 Cooper Avenue**
**Upper Montclair New Jersey 07043 (US)**
Inventor: **Girijavallabhan, Viyyoor Moopil**
**905 Broad Street Fairway Garden Apartment G7**
**Bloomfield New Jersey 07003 (US)**
Inventor: **Cavender, Patricia Lee**
**54 Rocky Brook Drive**
**Cranbury New Jersey 08512 (US)**
Inventor: **Sarre, Olga**
**12 Valhalla Way**
**Verona New Jersey 07044 (US)**
Inventor: **McCombie, Stuart Walter**
**159 Pleasant Valley Way**
**West Orange New Jersey 07052 (US)**

(74) Representative: **Antony, Fritz, Dr. et al,**
**P.O. Box 601 Winkelriedstrasse 35**
**CH-6002 Lucerne (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 005 889 B1

Courier Press, Leamington Spa, England.

# Process for the preparation of beta-lactam derivatives, the novel derivatives so obtainable and pharmaceutical compositions containing them

This invention relates to a process for the preparation of β-lactam derivatives, especially of derivatives of β-lactam antibiotics, to the novel derivatives so obtainable and to pharmaceutical compositions containing them. More specifically, this invention relates to a novel process for preparing such β-lactam derivatives having a substituted hydroxymethyl group at the carbon atom α to the lactam carbonyl group and to novel derivatives so obtainable.

DiNinno *et. al.* disclose in *J. Org. Chem. 42* pp. 2960—2965 a process for introducing a specified substituted hydroxymethyl group at the carbon atom α to the lactam carbonyl group in a particular penicillanate. That process comprises a two-step process in which a solution of benzyl 6,6-dibromopencillanate is treated with n-butyllithium or methyl magnesium bromide and an excess of acetaldehyde at temperatures of —70°C and below to give a 6-bromo-6-(α-hydroxyethyl)-intermediate which must then be treated with a zinc-silver couple to remove the 6-bromo atom. Mention is made of the direct application of the process to a benzyl-6α-bromo (and 6α-iodo)-penicillanate starting material to give the desired product in one step but it is noted that such could be obtained only in very low yield. The process is further described by DiNinno as applied to the preparation of a corresponding tertiary-butyl 7-(α-hydroxyethyl)-cephalosporanate with similar results.

We have now found that a substituted hydroxymethyl group may be introduced into a β-lactam starting material at a position α to the lactam carbonyl group in effectively one step and in acceptable yield, without the need to employ unduly low temperatures.

In its process aspect, the present invention provides a process for the preparation of a β-lactam derivative having a substituted-hydroxymethyl group at the carbon atom α to the lactam carbonyl group which process comprises reacting in an anhydrous aprotic medium an α-halo-β-lactam starting material with activated zinc or zinc amalgam in the presence of an appropriate aldehyde or ketone and then hydrolysing the resulting zinc complex.

The use of activated zinc in the replacement of a halogen-carbon bond by a carbon-carbon bond is known in the Reformatski reaction, where an α-halo-ester is reacted, with activated zinc to give an intermediate which upon reaction with a carbonyl compound gives a zinc complex yielding upon hydrolysis a corresponding β-hydroxy-ester.

While the replacement of the α-halogen atom of a α-halo-ester with a carbon substituent by reaction of the α-halo-ester with activated zinc and an aldehyde or ketone is known, it is surprising that this reaction proceeds with α-halo-β-lactams, for it is also known that the halogen atom of an α-halo-β-lactam is relatively unreactive and that the β-lactam ring is prone to degradation. Thus while α-halo-esters in general readily react with an aldehyde or ketone in the presence of phosphine, β-lactams having a halogen atom at the α-position are unreactive under comparable conditions. Further while the treatment of an α-halo-ester with sodium azide gives rise to the corresponding azide, treatment of an α-halo-β-lactam results in degradation of the lactam ring.

The process for the introduction of a substituted hydroxymethyl group in accordance with the present invention is of general applicability to β-lactam antibiotics and other compounds containing the β-lactam ring system. The precise nature of the β-lactam starting material is not critical.

Thus while the process finds particular utility applied to β-lactams having the fundamental penam and cepham ring systems present in the penicillins and cephalosporins, namely the structures IV and V wherein the dotted line represents an optional double bond:

(IV) 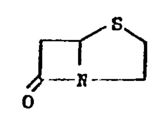 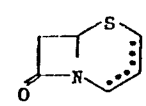 (V)

the process is widely applicable to other β-lactam ring systems. For instance the process may be applied to β-lactams having ring structures such as VI described in *J. Chem. Soc. C.*, 2093 (1969).

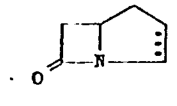 (VI)

wherein the dotted lines indicates the presence of an optional double bond; such as VII, described in *Can. J. Chem. 55*, 468 (1977), *Can. J. Chem. 55*, 484 (1977) and Belgian Patents 850,779 and 850,593

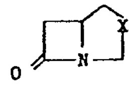 (VII)

wherein X is sulfur or oxygen;

such as VIII, described in U. S. Patents 4,068,075, 4,068,078, and 4,068,080

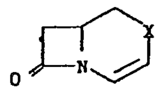

(VIII)

wherein X is nitrogen, sulfur or oxygen;
such as IX and X, described in Recent Advances in the *Chemistry of β-Lactam Antibiotics*, Special Publication No. 28, Chapter 28, p. 213 (1977),

(IX) 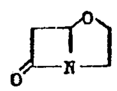 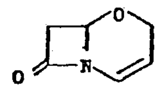 (X)

such as XI,

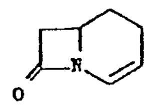

(XI)

such as XII, described in *J. Amer. Chem. Soc. 88*, 852 (1966),

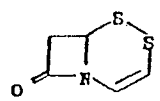

(XII)

such as XIII, described in *J. Amer. Chem. Soc. 88*, 852 (1966),

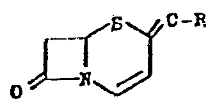

(XIII)

and those such as XIV,

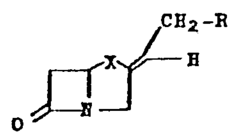

(XIV)

wherein X is nitrogen, oxygen or sulfur and R is hydroxy, acyl ester, cyanide, mercapto, S—$CH_3$, nitrogen function or halogen.

The process is also applicable to introducing a substituted hydroxymethyl group at the carbon atom $\alpha$ to the lactam carbonyl group of a β-lactam having the substituted ring system:

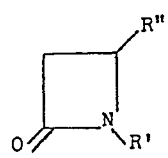

where R′ and R″ may be broadly defined and for R′ include substituted or unsubstituted hydrocarbyl groups of which alkenyl may be specifically mentioned and for R″ include substituted or unsubstituted hydrocarbyl groups and, in particular, alkoxy.

Functional groups in the α-halo-β-lactam starting material which preferentially react with the activated zinc or zinc amalgam or give rise to other undesirable side reactions or which have a substituent generating a proton (for instance a salified ammonium function) should be suitably protected. Examples of such groups are carboxy, amino and hydroxy groups and examples of specific protecting groups which may be used are benzyl and benzyloxy. Such groups as cyano, alkyl and alkoxy are stable to the reaction conditions and thus may be present in the halo-β-lactam molecule without protection.

As previously stated the process of the present invention finds particular utility applied to β-lactams of the penicillin and cephalosporin type. Here the α-halo-β-lactam starting materials are represented by the basic formulae (II) and (III):

(II) 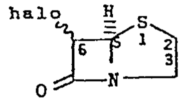 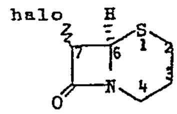 (III)

in which halo represents chloro, iodo and preferably bromo and the wavy line indicates either the $\alpha$ or $\beta$-stereochemical configuration at the 6 or 7 position.

The 6- or 7-halo starting material of the penicillin or cephalosporin type may be prepared from the appropriately substituted 6-amino- or 7-amino-$\beta$-lactam by reaction of the 6-amino- or 7-amino-$\beta$-lactam with sodium nitrite and a suitable source of halide under the conditions described in *J. Org. Chem. 27* 2668 (1962). Alternatively, these starting materials can be prepared from the 6- or 7-diazo compound by reaction with the appropriate hydrogen halide. Additional methodology for their preparation is described in Flynn, "Cephalosporins and Penicillins", pp. 101—105, Academic Press (1972). Other $\alpha$-halo-$\beta$-lactam starting materials may be similarly prepared.

As examples of starting materials giving rise to particularly useful end products there may be mentioned:

a) 6-halopenicillanic acid derivatives of the general formula (XV)

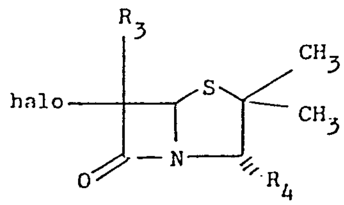 (XV)

wherein halo is a halogen atom selected from chlorine, bromine or iodine, $R_3$ is hydrogen or lower alkoxy, and $R_4$ is cyano or $COOR_{11}$ wherein $R_{11}$ is a readily removable ester-forming moiety, the term "readily removable ester-forming moiety" as used herein meaning commonly employed carboxylic acid protecting groups employed for protecting the $C_3$ carboxylic acid group of penicillins and the corresponding $C_4$ carboxylic acid group of the cephalosporins. Representative of such groups are *t*-butyl, benzyl, benzhydryl, *p*-nitrobenzyl, 4-methoxybenzyl, 3,5-dimethoxybenzyl and tetrahydropyranyl and the like cleavable ester moieties;

b) 7-halocephalosporanic acid esters of the formula XVI

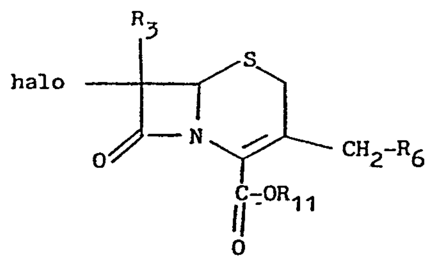 (XVI)

wherein halo, $R_3$ and $R_{11}$ are as defined for formula XV, $R_6$ is hydrogen, lower alkanoyloxy, lower alkoxy, lower alkylthio, pyridinium, or a group selected from

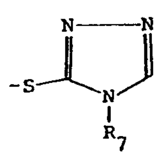 , 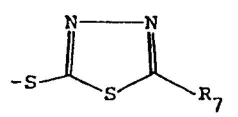 or 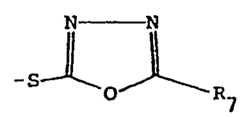

wherein $R_7$ is hydrogen, lower alkyl, phenyl, lower alkylphenyl, halophenyl, hydroxyphenyl, lower alkoxyphenyl, or

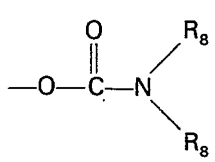

wherein $R_8$ represents hydrogen or lower alkyl;

c) 6-halopenicillanic acid esters of the general formula XVII

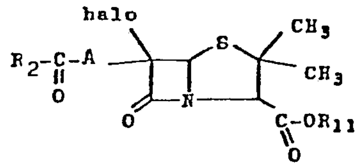

(XVII)

wherein halo and $R_{11}$ are as hereinbefore defined for formula XV,

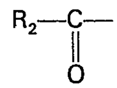

is an acyl group, and A is a group of the formula:

$$-(CH_2)_m-X_p-(CH_2)_n-$$

wherein X is O or S, m is 0—3, n is 0—3, p is 0—1 with the proviso that m + n + p is 3—5;

d) Compounds of the general formula XVIII

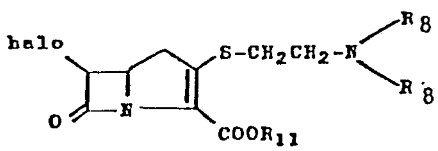

(XVIII)

wherein halo, $R_8$ and $R_{11}$ are as defined for formula XVI; and

e) compounds of the general formula XIX

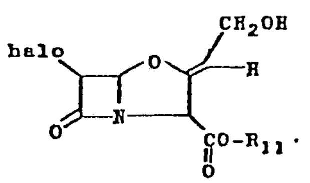

(XIX)

wherein halo and $R_{11}$ are as defined for formula XV.

In the process of the present invention any suitable aldehyde or ketone may be employed its precise nature not being critical except, of course, that the aldehyde or ketone should not contain functional groups which preferentially react with the activated zinc or the zinc-halo-$\beta$-lactam intermediate or give rise to other undesirable side reactions or which have a substituent generating a proton (for instance a salified ammonium function). Thus where the aldehyde or ketone contains a hydroxy or amino group these groups should be protected in known manner using suitable protecting groups of which benzyl, benzhydryl and tetrahydropyranyl groups may be mentioned as specific examples.

The aldehyde and ketone reactants may be aliphatic, alicyclic, aromatic or heterocyclic, an aldehyde or ketone representable by the general formula $R'_1$—CO—$R'_2$ giving rise to $\beta$-lactam derivative having at the carbon atom $\alpha$ to the lactam carbonyl group a corresponding substituted hydroxymethyl group of the formula

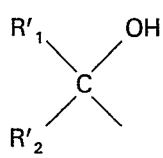

Suitable aliphatic aldehydes and ketones are those where $R'_1$ and/or $R'_2$ are aliphatic hydrocarbyl groupings having 1 to 20 carbon atoms including saturated and unsaturated groupings and straight and branched-chain groupings. The aliphatic hydrocarbyl moiety may also be a cycloalkyl group containing 4 to 7 carbon atoms. The ketone may also be an alicyclic ketone such as cyclopentanone, cyclohexanone or cycloheptanone. In view of the nature of the end-products obtained, aromatic aldehydes or ketones and in particular substituted benzaldehydes are particularly useful in the process of the present invention. Suitable heterocyclic carbonyl compounds are those having a tetrahydrofuryl or thienyl substituent.

The carbonyl group of the aldehyde or ketone may also be in a side chain extending from the ring

system including the $\beta$-lactam and in particular substituting the $\alpha$-carbon atom thereof.

The zinc used in the process of the present invention may be activated in conventional manner. Typical methods for accomplishing the activation are described in: Fieser and Fieser, *Reagents for Organic Synthesis*, Vol. 1, p. 1269 (1967); *Chem. Communications*, 269 (1973); *Synthesis*, 452 (1975) and *"Accounts of Chemical Research"*, Vol. 10, 301 (1977). Where used, zinc amalgam may be prepared in conventional manner.

The process of the present invention is effected in an anhydrous aprotic medium. Anhydrous conditions are necessary to avoid converting the zinc-halo-$\beta$-lactam intermediate into the corresponding dehalo-$\beta$-lactam.

The reaction medium may suitably comprise a polar or non-polar aprotic organic solvent in which the halo-$\beta$-lactam starting material is soluble. Particularly useful solvents for the process are ethers such as ethyl ether, tetrahydrofuran, dioxan and diglyme; aromatic hydrocarbons such as benzene, toluene, xylene; and tertiary amides such as dimethylformamide and diethylformamide. Benzene and tetrahydrofuran are especially preferred solvents.

Where the aldehyde or ketone is a liquid in which the halo-$\beta$-lactam is soluble, then the carbonyl compound itself may suitably serve as the reaction medium.

The process of the present invention may advantageously be carried out under an inert atmosphere such as under a nitrogen or argon atmosphere particularly where undesirable side reactions may otherwise take place. Unless the carbonyl reactant is gaseous, the reaction may suitably be carried out under atmospheric pressure.

With the process of the present invention, it is not necessary to employ inconveniently low temperatures. Suitably, the reaction may be effected at temperatures within the range of about 0°C to 110°C, and generally at room temperature. Alternatively, the reaction may be effected at the reflux temperature of the reaction medium bearing in mind that temperatures above the boiling point of the aldehyde or ketone should be avoided. The reaction time will be dependent upon the nature of the reactants and the reaction temperature employed. The progress of the reaction can conveniently be followed by thin layer chromatography or other known techniques. The reaction time may typically be in the range of from 2 to 12 hours.

Hydrolysis of the resulting zinc complex may be effected by treating it with an aqueous medium, preferably at a pH of about 5 to 7, that is a pH from 4 to 8 most preferably a pH of 4 to 7. For instance the zinc complex may be treated merely with distilled water or more preferably with an aqueous solution buffered to the required pH most preferably to a pH of 5. The required hydrolysis is in general very readily effected such as by merely washing a solution of the zinc complex with the aqueous medium.

The desired product obtained by the process of the invention may be isolated using known techniques and then separated into its stereochemical isomers in manner known per se such as by chromatographic separation.

While it is not intended that the process of the present invention be limited by any particular explanation of the reaction mechanism, it is believed that the overall process proceeds through a zinc-halo-$\beta$-lactam intermediate, this intermediate then reacting with the aldehyde or ketone to form the zinc complex which is then hydrolysed as schemicatically represented by:

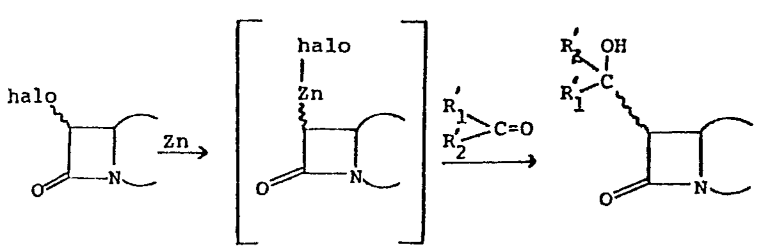


wherein

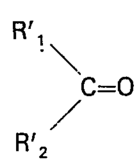

is the aldehyde or ketone reactant and halo is chloro, bromo or iodo.

The aldehyde or ketone may also form part of the $\beta$-lactam molecule and here the overall reaction can be schematically represented by:

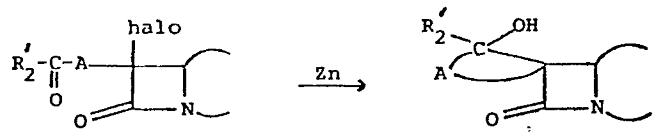

where $R'_2CO$— is an acyl group and A is a group of the formula:

$$-(CH_2)_m-X_p-(CH_2)_n-$$

as previously defined.

In the process of the present invention an excess of the zinc and an excess of the ketone or aldehyde reactants is typically employed, for instance the number of equivalents of zinc per mol of $\alpha$-halo-$\beta$-lactam starting material being usually greater than 10:1 and the mol ratio of carbonyl reactant to $\alpha$-halo-$\beta$-lactam starting material being usually greater than 5:1. The precise excess of the zinc and carbonyl reactants is not considered critical however.

The product of the process of this invention, namely the appropriate $\beta$-lactam derivative bearing a substituted hydroxymethyl group at the carbon atom $\alpha$ to the lactam carbonyl group may be subjected, if desired, to various finishing steps including those well known in the field of penicillin and cephalosporin chemistry.

Thus, any carboxylic acid group may be converted in known manner into a pharmaceutically acceptable cationic salt such as an alkali metal salt of which the sodium salts may be specifically mentioned. For instance, 6$\beta$-(1-hydroxyethyl)-penicillanic acid is converted into the corresponding sodium salt by treatment with 1 equivalent of sodium bicarbonate in water. Further, any suitable basic grouping such as a tertiary nitrogen function may be similarly converted in known manner into a pharmaceutically acceptable acid addition salt of which the hydrochloride salts may be specifically mentioned.

Further, where the end product has a functional group which has been protected during the reaction, for instance a protected carboxy, amino or hydroxy group then that protecting group may be removed. Thus any carboxylic ester grouping may, if desired, be hydrolysed. For instance the ester group designated —$COOR_{11}$ in the products obtained from the starting materials XV to XIX may be subjected to reductive cleavage by hydrogenation in the presence of a palladium or palladium-on-carbon catalyst. Typical times are in the range of 6—48 hours and typical pressures in the range of atmospheric to 4.2 kg.cm—2. For example, benzyl-6$\beta$-($\alpha$-hydroxy-*o*-fluorobenzyl)penicillanate is converted to 6$\beta$-($\alpha$-hydroxy-*o*-fluorobenzyl)penicillanic acid by hydrogenation for 40 hours at atmospheric pressure in the presence of a 30% palladium-on-carbon catalyst.

The hydroxy group in the substituted hydroxymethyl moiety of the end products of the process of the present invention may be etherified or esterified in manner known per se, for instance esterified to give the corresponding formyloxy or alkanoyloxy derivative. This may be achieved using an appropriate acid chloride, acid anhydride or activated amide in the presence of an acid acceptor. The acid acceptor may be an organic base such as pyridine or triethylamine or an inorganic base such as sodium or potassium hydroxide, or sodium or potassium bicarbonate. For instance, a 6-(1-hydroxyethyl)-penicillanate may be converted to a 6-(1-formyloxyethyl)penicillanate using dimethylformamide and tosyl chlorides or methanesulfonyl chloride; or to a 6-(1-acetoxyethyl)penicillanate using acetic anhydride or acetylchloride and pyridine.

Use of an alkylsulfonyl halide or alkylsulfonic anhydride in the above procedure in the presence of an acid acceptor gives rise to the corresponding alkylsulfonyloxy derivative. The alkylsulfonyloxy group may in turn be converted into a halo substituent by contacting with the appropriate halide ion. For instance sodium iodide may be used to convert benzhydryl (6-(1-methanesulfonyloxyethyl) penicillanate to the corresponding benzhydryl (6-(1-iodo-ethyl)penicillanate.

Where a cephalosporin product having a methylene group at the 3-position is obtained, the methylene double bond may be isomerised by base to afford a mixture of the $\Delta^2$ and $\Delta^3$ cephems. This mixture may then be separated by chromatography in standard manner to give the $\Delta^3$ compound in a pure state.

Alternatively, the mixture is first oxidized to the sulfoxide according to the method of O'Connor and Lyness, *J. Amer. Chem. Soc. 86*, 3840 (1964). The sulfoxide is then reduced in the presence of an activating agent according to the method of Kaiser, *et. al., J. Org. Chem. 35*, 2430 (1970) to afford the $\Delta^3$ compounds in their pure state.

The process of the present invention gives rise to a group of novel $\beta$-lactam derivatives bearing on the carbon atom $\alpha$ to the lactam carbonyl group a specified substituted hydroxymethyl group.

In U.S.P. 4,076,826 and U.S.P. 4,078,068 there are disclosed specified 2-substituted clavulanic acids bearing a 6-hydroxyethyl substituent which are indicated as exhibiting useful antibacterial activity. Similarly, DOS 2,751,624 discloses specified 1-carba-2-penem-3-carboxylic acid derivatives having, inter alia, at the 6-position a hydroxyethyl group and these compounds are indicated in similar terms as exhibiting useful antibacterial activity.

We have now surprisingly found that the novel group of $\beta$-lactam derivatives of the present invention while possessing useful antibacterial activity also exhibit useful activity where penicillinase or cephalosporinase are present.

Thus, in another of its aspects the present invention provides novel $\beta$-lactam derivatives of the general formula I

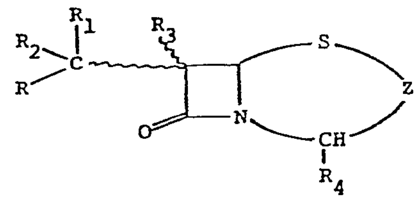

(I)

and the pharmaceutically acceptable salts thereof.
wherein Z represents:

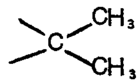 , 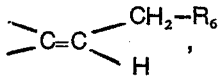 , 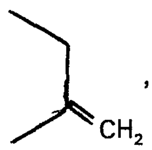 , or 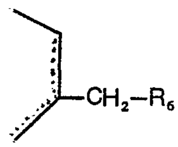

where the dotted line represents an optional double bond and $R_6$ represents hydrogen, lower alkanoyloxy, lower alkoxy, lower alkylthio, pyridinium or a group

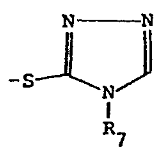 , 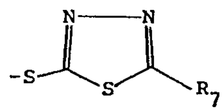 or 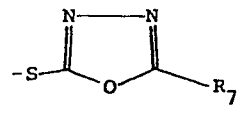

where $R_7$ is hydrogen, lower alkyl, phenyl, lower alkylphenyl, halophenyl, hydroxyphenyl, lower alkoxyphenyl or $R_7$ represents the group

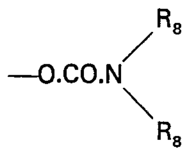

where each $R_8$ represents hydrogen or lower alkyl,

R represents OH, lower alkoxy, formyloxy, lower alkanoyloxy, lower alkylsulfonyloxy or halogen,

$R_1$ and $R_2$ are groupings corresponding to those in the aldehyde or ketone $R_1$.CO.$R_2$ employable in the process of the present invention, preferably:

$R_1$ representing lower alkyl, lower alkenyl, lower alkynyl, lower cycloalkyl or a heterocyclic group containing an oxygen or sulfur heteroatom all of which groupings may be substituted by a free or, protected-amino.-hydroxy or- carboxyl group, or more preferably $R_1$ represents substituted phenyl, as hereinafter specified.

$R_2$ represents hydrogen or is as defined for $R_1$,

$R_3$ represents hydrogen or lower alkoxy or $R_3$ together with $R_1$ represents a divalent grouping A of the formula

$$-(CH_2)_m-(X)_p-(CH_2)n-$$

in which grouping X is O or S, m and n independently are each O or an integer from 1 to 3, p is O or 1 with the proviso that m+n+p is 3, 4 or 5, and

$R_4$ represents cyano, a carboxylic acid group COOH or an ester thereof $-COOR_{11}$ where $R_{11}$ is a readily removable ester-forming moiety, with the proviso that when Z is

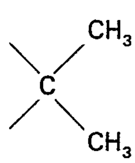 or 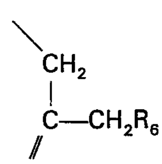

R is OH, $R_2$ and $R_3$ are hydrogen and $R_1$ is $CH_3$, then $R_4$ is —CN or a carboxylic acid ester grouping other than t-butyl or benzyl.

As used herein the term "lower alkyl" means alkyl groups containing up to 6 carbon atoms such as methyl, ethyl, propyl, butyl, pentyl and hexyl as well as the corresponding branched-chain isomers thereof. The terms "lower alkenyl" and "lower alkynyl" mean the corresponding unsaturated groups containing up to 6 carbon atoms. By "lower cycloalkyl" is meant such groups containing 4 to 7 carbon atoms.

By "lower alkoxy" is meant groups containing 1—6 carbon atoms such as methoxy, ethoxy and propoxy while by "lower alkanoyl" is meant groups containing 2 to 7 carbon atoms such as acetyl, propionyl, all the aforementioned definitions including straight and branched chain isomers. The term "lower alkanoyloxy" is likewise defined.

Unless otherwise specified "halogen" or "halo" encompasses fluorine, chlorine, bromine and iodine. "Lower alkylthio" includes methylthio, ethylthio, *n*-propylthio and isopropylthio, while "loweralkylphenyl" means a phenyl group substituted by one or two alkyl groups having up to 6 carbon atoms.

As used herein, the term "halophenyl" refers to mono- and dihalosubstituted phenyl groups such as mono- and dichlorophenyl, mono- and difluorophenyl and mono- and dibromophenyl groups.

The term "substituted phenyl" as used herein refers to phenyl substituted by one or more substituent groups selected from halo (that is chloro, bromo and fluoro), nitro, lower alkyl, lower alkenyl, lower alkynyl, lower alkoxy, and free and protected-amino,- hydroxy and -carboxy groups. Exemplary of such aryl groups are 3,4-dichlorophenyl, 2-fluorophenyl, 3-nitrophenyl, 4-methylphenyl, 4-ethoxyphenyl, 2,6-dimethoxyphenyl, 3- and 4-aminophenyl, 4-hydroxyphenyl, 4-carboxyphenyl and in particular 4-aminomethylphenyl, 4-dimethylaminophenyl, 3-benzyloxyphenyl, 4-(2'-tetrahydropyranyloxy)phenyl, 4-phenylmethoxycarbonylphenyl and diphenylmethoxycarbonylphenyl.

The compounds of the general formula I have the fundamental penam and cepham ring system present in the penicillin and cephalosporin antibiotics.

A preferred value for R is hydroxy and for $R_3$ is hydrogen. While $R_1$ and $R_2$ can represent a wide variety of substituents, their values merely depending upon the aldehyde or ketone from which their groups are derived, it may be said that $R_2$ is preferably hydrogen and $R_1$ preferably an aryl group as herein defined such as an N-substituted or unsubstituted *p*-amino-phenyl group, a free or protected *p*-carboxy-phenyl group, a free or protected *o*- or *p*-hydroxy-phenyl.

As previously mentioned the $\beta$-lactam derivatives of the present invention exhibit useful antibacterial activity. Moreover, representative derivatives tested surprisingly exhibit useful activity in the presence of such enzymes as penicillanase and cephalosporinase that is the derivatives act as penicillanase and cephalosporinase inhibitors.

This may be illustrated by reference to the results obtained for the following compounds:

Compound A:— 6$\beta$-(*p*-benzyloxy-$\alpha$-hydroxybenzyl)-2,2-dimethyl-3$\alpha$-cyanopenam.

Compound B:— 6$\alpha$-(*p*-benzyloxy-$\alpha$-hydroxybenzyl)-2,2-dimethyl-3$\alpha$-cyanopenam.

Compound C:— 6$\beta$-(*p*-diphenylmethoxycarbonyl-$\alpha$-hydroxybenzyl)-2,2-dimethyl-3$\alpha$-cyanopenam.

The above compounds were tested for antibacterial activity against specified organisms, including antibacterial activity in the presence of penicillanase and cephalosporinase. The results expressed in terms of minimum inhibitory concentrations (MIC values) are given in Table I (without penicillanase and cephalosporinase) and Table II (in the presence of penicillinase and cephalosporinase).

TABLE I

| | *Staphylococcus Aureus* MIC/mcg/ml | | *Enterococcus* MIC/mcg/ml | |
|---|---|---|---|---|
| Compound | 24 hours | 48 hours | 24 hours | 48 hours |
| A | .125—8 | 4—64 | 2—4 | >64 |
| B | 18—16 | 16—64 | 1—2 | 4—8 |
| C | 32—64 | 64 | 2—32 | >64 |

TABLE II

| | *Staph. epidermis 27626* (contains penicillinase) MIC/mcg/ml | | *B. subtilis 1119601* (contains cephalosporinase) MIC/mcg/ml | |
|---|---|---|---|---|
| Compound | 24 hours | 48 hours | 24 hours | 48 hours |
| A | .25 | 2 | 8 | 16 |
| B | 2 | 4 | >64 | — |
| C | .25 | 2 | 8 | 16 |

In general it may be said that the compounds of the present invention have MIC values in the range of 0.1 to 100 mcg/ml when tested against such organisms as *Staphylococcus* epidermidis, *Salmonella, Bacillus* subtilis, and *Pseudomonas* aeruginosa including where enzymes such as penicillinase and cephalosporinase are present.

The compounds of the present invention may be formulated for oral, intramuscular or intravenous therapy.

Thus, the present invention includes within its scope pharmaceutical compositions comprising the β-lactams of this invention with a compatible pharmaceutical carrier therefor.

The dosage administered of the β-lactams of this invention is dependent upon the age and weight of the animal species being treated, the mode of administration, and the type and severity of bacterial infection being prevented or reduced. Typically, the dosage administered per day will be in the range of 100—5000 mg with 500—1000 mg being preferred. For the preferred compounds of the invention tested, it is considered that the compounds are effectively non-toxic at the dose-rates indicated for treatment of a bacterial infection.

Included within the preferred compounds of the invention are

6α- and 6β-(p-benzyloxy-α-hydroxybenzyl)-2,2-dimethyl-3α-cyanopenam, and

6α- and 6β-(*p*-diphenylmethoxycarbonyl-α-hydroxy-benzyl)-2,2-dimethyl-3α-cyano-penam.

For oral administration, the compounds of this invention may be formulated in the form of tablets, capsules, elixirs or the like. For parenteral administration they may be formulated into solutions or suspensions for intramuscular injection.

The invention will now be illustrated by way of the following, in which Examples 1—24 illustrate the preparation of compounds in accordance with the invention and Formulation Examples 1—5 the preparation of pharmaceutical compositions.

Example 1

To a solution of 500 mg methyl 6α-bromopenicillanate in 7 ml anhydrous acetaldehyde is added active zinc dust with constant stirring at 20°C. The progress of the reaction is monitored by thin layer chromatography using a 10% acetone in benzene solvent system. Upon completion of the reaction, the zinc is removed by filtration, and the mixture is diluted with 50 ml ethyl acetate and washed with phosphate buffer (10 ml) of pH 5 to remove the zinc salts. After drying the solution over anhydrous sodium sulfate and removing the solvent under vacuum, the product is chromatographed through silica using 3% acetone in benzene as eluant. The initial fraction is discarded. The following fractions afford, in 80:20 ratio, methyl 6β-(1-hydroxyethyl)penicillanate and methyl 6α-(1-hydroxyethyl)-penicillanate. The 6β compound has the following formula

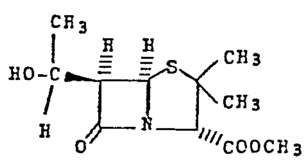

These 6β and 6α compounds had the following mass spectrum, nuclear magnetic resonance and infra-red data:

6β Compound

MS: 259 (M+)

NMR: 5.4 (d, J = 5Hz, 1H); 4.4 (s, 1H); 4.22 (m, 1H); 3.75 (s, 3H); 3.5 (dd, J = 5 and 6, 1H); 2.8 (OH); 1.63 (s, 3H); 1.4 (s, 3H); 1.18 (d, H = J, 3H)

IR: 3,550, 1770, 1740 cm.$^{-1}$

6α Compound

NMR: 5.25 (d, J = 1.5Hz, 1H); 4.45 (s, 1H); 4.25 (m, 1H); 3.75 (s, 3H); 3.35 (dd, J = 1.5 and 6, 1H); 2.5 (OH); 1.63 (s, 3H); 1.4 (s, 3H); 1.25 (d, J = 6, 3H)

IR: 3500, 1770, 1740 cm.$^{-1}$

Example 2

A solution of 500 mg methyl 6α-bromopenicillanate in 25 ml dry benzene containing 1 ml benzaldehyde and 2 g active zinc dust is refluxed under a nitrogen atmosphere with vigorous stirring for approximately 6 hours. Upon termination of the refluxing, the reaction mixture is diluted with 50 ml ethyl acetate and washed with a phosphate buffer of pH 5. The solution is dried over anhydrous sodium sulfate and the solvents are removed under vacuum. The products were separated by preparative thin layer chromatography using multiple elutions with 3% acetone in chloroform to afford, in a 6:1 ratio, methyl 6α-(α-hydroxybenzyl)penicillanate and methyl 6β-(α-hydroxybenzyl)penicillanate having the following spectral data:

6$\alpha$ Compound
NMR: 7.5—7.3 (5H); 5.35 (d, J = 1.5Hz, 1H); 5.2 (m, 1H); 4.35 (s, 1H); 3.7 (s, 3H); 3.45 (1H); 1.75 (s, 3H); 1.4 (s, 3H)
IR: 3500, 1775, 1740, 1600 cm.$^{-1}$

6$\beta$ Compound
NMR: 7.5—7.3 (5H); 5.2 (2H); 4.4 (s, 1H); 3.7 (s, 3H); 3.4 (1H); 1.6 (s, 3H); 1.4 (s, 3H)
IR: 3500, 1780, 1740, 1605 cm.$^{-1}$

Example 3

To a solution of 8 g benzhydryl 6$\alpha$-bromopenicillanate in 150 ml dry tetrahydrofuran is added 35 ml dry acetaldehyde, followed by 13 g active zinc. The mixture is then stirred very vigorously for 2½ hours. Upon completion of the reaction, the mixture is taken in 500 ml ethyl ether and washed with phosphate buffer of pH 5. The solution is dried over anhydrous sodium sulfate and the solvents are removed under vacuum. The resultant foamy solid is chromatographed through silica gel using 5% acetone in benzene to afford a major amount of benzhydryl 6$\beta$-(1-hydroxyethyl)penicillanate and a minor amount of benzhydryl 6$\alpha$-(1-hydroxyethyl)penicillanate having the following spectral data:

6$\beta$ Compound
MS: 411 ($M^+$)
NMR: 7.4 (10H); 7 (s, 1H); 5.45 (d, 1H); 4.6 (s, 1H); 3.6 (dd, 1H); 2.8 (OH); 1.7 (s, 3H); 1.4 (3H),
IR: 3500, 1770, 1740 cm.$^{-1}$

6$\alpha$ Compound
MS: 411 ($M^+$)
NMR: 7.4 (10H); 7.05 (s, 1H); 5.3 (d, J = 1.5, 1H); 4.55 (s, 1H); 4.2 (m, 1H); 3.4 (dd, J = 1.5 and 6, 1H); 2.0 (OH); 1.65 (s, 3H); 1.4 (d, 3H); 1.35 (s, 3H)
IR: 3500, 1770, 1740 cm.$^{-1}$

Example 4

To a solution of 1.5 g benzhydryl 6$\alpha$-bromopenicillanate in 20 ml dry tetrahydrofuran is added 3 ml *o*-fluorobenzaldehyde, followed by 2.5 g active zinc. After stirring vigorously for 4 hours, the zinc is filtered off from the reaction mixture and the filtrate is taken up in 200 ml ethyl acetate and washed first with phosphate buffer of pH 5 and then with 15 ml of 1% aqueous sodium bicarbonate. After drying the solution over anhydrous sodium sulfate and removal of the solvents under vacuum, the product is chromatographed utilizing 5% acetone in toluene to afford benzhydryl 6$\beta$-(*o*-fluoro-$\alpha$-hydroxybenzyl)-penicillanate and benzhydryl 6$\alpha$-(*o*-fluoro-$\alpha$-hydroxybenzyl)-penicillanate. The 6$\beta$ compound has the following spectral data:

MS: 473, 324, 167
NMR: 7.6—6.8 (14H); 7.0 (s, 1H); 5.4 (d, J = 6Hz, 1H); 5.3 (d, J = 4.5Hz, 1H); 4.6 (s, 1H); 4.05 (dd, J = 4.5 and 6, 1H); 2.8 (OH); 1.65 (s, 3H); 1.2 (s, 3H)
IR: 3500, 1770, 1745 cm.$^{-1}$

When *p*-dimethylaminobenzaldehyde is substituted for the *o*-fluorobenzaldehyde in the above procedure, there is obtained benzhydryl 6-(*p*-dimethylamino-$\alpha$-hydroxybenzyl) penicillanate.

Example 5

To a solution of 2.5 g benzhydryl 6$\alpha$-bromopenicillanate in 35 ml dry tetrahydrofuran is added 5 ml cinnamaldehyde and 5.2 g active zinc. The resultant solution is stirred vigorously for 12 hours in the presence of a catalytic amount of copper (II) chloride. The mixture is then taken up in 200 ml ethyl acetate and washed with phosphate buffer of pH 5. The solution is dried over anhydrous sodium sulfate and the solvents removed under vacuum. The excess cinnamaldehyde is removed by using high vacuum and the product is chromatographed using 5% acetone in benzene to afford benzhydryl 6$\beta$-(1-hydroxy-3-phenylprop-2-enyl) penicillanate and benzhydryl 6$\alpha$-(1-hydroxy-3-phenylprop-2-enyl)-penicillanate. The 6$\beta$ and 6$\alpha$ compounds had the following spectral data:

6$\beta$ Compound
MS: 499 ($M^+$)
NMR: 7.4—7 (16H); 6.5—5.8 (2H); 5.35 (d, J = 4.5, 1H); 4.8 (m, 1H); 3.65 (dd, 1H); 2.6 (OH); 1.65 (s, 3H); 1.2 (s, 3H); 4.5 (s, 1H)
IR: 3500, 1770, 1745 cm.$^{-1}$ ($CHCl_3$ solution).

6$\alpha$ Compound
NMR: 7.4—7 (16H); 6.8—6.05 (2H); 5.4 (1H); 4.75 (1H); 4.65 (s, 1H); 3.6 (1H); 2.6 (OH); 1.6 (s, 3H); 1.25 (s, 3H)
IR: 3500, 1770, 1745 cm.$^{-1}$ ($CHCl_3$ solution).

Example 6

A solution of 1 g of benzyl 7$\alpha$-bromo-3-desacetoxymethyl-3-methylene-cephalosporanate in 50 ml dry tetrahydrofuran is stirred with 5 ml dry acetaldehyde and 5 g active zinc under a nitrogen atmosphere for 1.5 hours. The reaction mixture is then filtered, diluted with 100 ml ethyl acetate and washed with phosphate buffer of pH 5. The solution is dried over anhydrous sodium sulfate and the solvents removed under vacuum. NMR spectra show that the exocyclic methylene group is intact. Chromatography on thin layer chromatography plates using 5% acetone in benzene affords benzyl 7$\beta$-(1-hydroxyethyl)-3-desacetoxymethyl-3-methylenecephalosporanate and benzyl 7$\alpha$-(1-hydroxyethyl)-3-desacetoxymethyl-3-methylenecephalosporanate.

Each of the two compounds is then independently treated with 500 mg triethylamine in 20 ml ethyl acetate to shift the double bond to conjugation. The solvents are removed under high vacuum to afford, respectively, benzyl 7$\beta$-(1-hydroxyethyl)desacetoxy-$\Delta^2$- or -$\Delta^3$-cephalosporanate and benzyl 7$\alpha$-(1-hydroxyethyl)desacetoxy-$\Delta^2$- or -$\Delta^3$-cephalosporanate, having the following spectral data:

7$\beta$ Compound ($\Delta^3$)
MS: 333 ($M^+$)
NMR: 7.4 (5H); 5.3 (s, 2H); 4.9 (d, J = 6, 1H); 4.4 (m, 1H); 3.7 (dd, 1H); 3.3 (2H); 2.05 (s, 3H); 1.35 (d, 3H)
IR: 3500, 1760, 1730 $cm.^{-1}$

7$\alpha$ Compound ($\Delta^3$)
NMR: 7.3 (5H); 5.2 (2H); 4.7 (d, J = 1.5, 1H); 4.1 (m); 3.5—3.0 (3H); 2.0 (s, 3H); 1.2 (d, 3H)
IR: 3500, 1760, 1730 $cm.^{-1}$

Example 7

A solution of 1.6 g benzhydryl 7$\alpha$-bromocephalosporanate in 50 ml dry tetrahydrofuran is stirred with 3.2 g active zinc dust and 8 ml acetaldehyde for 20 hours. The reaction mixture is then filtered, diluted with ethyl acetate and washed with phosphate buffer of pH 5. The solution is dried over anhydrous sodium sulfate and the solvents are removed under vacuum. Chromatography of the resultant product affords benzhydryl 7$\beta$- and 7$\alpha$-(1-hydroxyethyl)cephalosporanate as well as the corresponding 7$\beta$-$\Delta^2$ and 7$\alpha$-$\Delta^2$ compounds.

Example 8

To a solution of 3.0 g benzyl 6$\beta$-bromo-6$\alpha$-methoxy-penicillanate in 35 ml dry tetrahydrofuran and 10 ml dry acetaldehyde is added 2 g zinc dust. The mixture is stirred at 25°C under a nitrogen atmosphere for 2 hours. The excess zinc is removed by filtration and washed with ethyl acetate, and the filtrates are washed first with dilute hydrochloric acid and then with sodium bicarbonate solution. After drying over anhydrous magnesium sulfate, the solvents are removed *in vacuo* and the residue chromatographed on silica gel, eluting with chloroform containing 0.5% methanol. The appropriate fractions containing the desired product ($R_f$ = 0.5 on silica gel in 1% methanol-chloroform) are combined and rechromatographed on silica using 7% ethyl acetate in benzene. Removal of the solvents and recrystallization from ether-hexane give the desired product, benzyl 6-(1-hydroxyethyl)-6-methoxy-penicillanate as a white solid melting at 86—88°C.

Example 9

To a solution of 0.3 g 2,2-dimethyl-3$\alpha$-cyano-6$\alpha$-bromopenam in 6 ml anhydrous tetrahydrofuran and 1.68 ml anhydrous acetaldehyde is added 1.0 active zinc dust. The reaction mixture is stirred vigorously at room temperature for 4 hours whereupon 1 ml phosphate buffer of pH 5 is added. After stirring a few minutes, the reaction mixture is diluted with ethyl acetate, filtered and the organic phase washed once with brine. After drying over anhydrous sodium sulfate, filtering and evaporating, the residue is chromatographed on silica gel, using 3% acetone in benzene as eluant. Thus isolated, in a 2:1 ratio, are 2,2-dimethyl-3$\alpha$-cyano-6$\beta$-(1-hydroxyethyl)penam and 2,2-dimethyl-3$\alpha$-cyano-6$\alpha$-(1-hydroxyethyl)penam. The 6$\beta$-compound has the following spectral data:

MS: 226 ($M^+$)
NMR: 1.23 (d, J = 6Hz); 1.60 (s); 1.73 (s); 3.50 (dd, J = 4.5 and 9 Hz); 4.20 (m); 4.60 (s); 5.27 (d, J = 4.5 Hz).

A by-product of this reaction, 2,2-dimethyl-3$\alpha$-cyanopenam, is also isolated in a minor amount.

Example 10

To a solution of 60 mg 2,2-dimethyl-3$\alpha$-cyano-6$\alpha$-bromopenam and 3 drops benzaldehyde in 1 ml dry tetrahydrofuran is added 1 g zinc dust. The reaction mixture is stirred at room temperature for about 12 hours under nitrogen. A few drops of phosphate buffer of pH 5 are added and then the mixture is diluted with ethyl acetate. The zinc dust is filtered off and washed with ethyl acetate. The ethyl

acetate extracts are combined, washed with aqueous sodium chloride, dried over anhydrous sodium sulfate, filtered and evaporated. Chromatography of the resulting mixture on silica gel using 10% acetone in chloroform affords, in an 11:6 ratio, 2,2-dimethyl-3α-cyano-6α-(α-hydroxybenzyl)penam and 2,2-dimethyl-3α-cyano-6β-(α-hydroxybenzyl)penam having the following spectral data:

6α Compound
NMR: 1.56 (s); 1.7 (s); 3.67 (m); 4.67 (s); 5.05, 5.18 (d, J = 6Hz); 5.18, 5.35 (d, J = 2.5Hz).

6β Compound
NMR: 1.698 (s); 1.72 (s); 3.92 (dd, J = 5 and 10Hz); 4.62 (s); 5.13 (d, J = 10Hz); 5.15 (d, J = 5Hz).

Example 11

A solution of 100 mg 2,2-dimethyl-3α-cyano-6α-bromopenam in 40 ml dry tetrahydrofuran is stirred with 60 mg *p*-dimethylaminobenzaldehyde and 500 mg active zinc for about 12 hours at room temperature. The reaction mixture is then diluted with ethyl acetate, washed with aqueous sodium chloride, dried over anhydrous sodium sulfate, filtered and evaporated. Chromatography of the resulting mixture on silica gel affords, in 11:1 ratio, 2,2-dimethyl-3α-cyano-6α-(*p*-dimethylamino-α-hydroxybenzyl)penam and 2,2-dimethyl-3α-cyano-6β-(*p*-dimethylamino-α-hydroxybenzyl)penam having the following special data:

6α Compound
NMR: 1.58 (s); 1.73 (s); 2.96; 3.67 (m); 4.62 (two s); 4.90, 5.03 (d, J = 6Hz); 5.08, 5.30 (d, J = 2.5Hz).

6β Compound
NMR: 1.68 (s); 1.71 (s); 2.94; 3.97 (dd, J = 5 and 10Hz); 4.6 (s); 5.0 (d, J = 10Hz); 5.10 (d, J = 5Hz).

Using 7α-bromo-4-cyano-3-cephem in the above procedure similarly affords 7α-(p-dimethylamino-α-hydroxybenzyl)-4-cyano-3-cephem.

Example 12

Using substantially the procedure detailed in Example 11, 2,2-dimethyl-3α-cyano-6α-bromopenam is reacted with *p*-benzyloxybenzaldehyde to afford 2,2-dimethyl-3α-cyano-6α-(*p*-benzyloxy-α-hydroxybenzyl)penam and 2,2-dimethyl-3α-cyano-6β-(*p*-benzyloxy-α-hydroxybenzyl)penam, having the following spectral data:

6α Compound
MS: 394 ($M^+$)
NMR: 7.6—7.1 (7H); 7—6.8 (d, J = 8, 2H); 5.3 and 5.05 (d, J = 1.5, 1H); 5.0 (s, 2H); 4.6 (s, 1H); 3.65 (m, 1H); 1.6 (s, 1H); 1.45 (s, 3H).

6β Compound
MS: 394 ($M^+$)
NMR: 7.6—7.2 (7H); 7.05—6.9 (d, J = 8, 1H); 5.05 (d, J = 4); 5.0 (s, 2H); 4.6 (s); 3.95 (dd, J = 4, 8, 1H); 2.8 (OH); 1.65—1.6 (2s, 6H).

Example 13

*Step A*

A solution of 11.0 g benzyl 6-diazopenicillanate 1β-oxide and 9 g 3-buten-1-ol in 120 ml dichloromethane is stirred with ice-cooling, and 7.5 g N-bromosuccinimide is added in portions over a 5 minute period. After stirring for 18 minutes at room temperature, the solution is washed successively with water, aqueous sodium sulfite and aqueous sodium bicarbonate. After drying over anhydrous magnesium sulfate and removal of the solvents *in vacuo*, the residue is chromatographed on silica gel by eluting rapidly with benzene followed by 10% ethyl acetate in benzene. The product, benzyl 6α-(3-buten-1-yloxy)-6β-bromopenicillanate 1β-oxide, is isolated as a yellow oil.

*Step B*

A solution of 9.2 g benzyl 6α-(3-buten-1-yloxy)-6β-bromopenicillanate 1β-oxide) (obtained in Step A) in 70 ml dry dimethylformamide is cooled to 5°C. Then, 2.4 ml phosphorus tribromide is added dropwise and the mixture is stirred for 1 hour at room temperature. The mixture is added to ice water and extracted with diethyl ether. The organic phase is separated and washed successively with water, sodium bicarbonate solution and saturated sodium chloride solution. After drying over anhydrous magnesium sulfate, the solvents are removed by evaporation. The crude product is chromatographed rapidly on 150 g silica gel using 1:1 dichloromethane-hexane as the eluant, affording benzyl 6β-bromo-6α-(3-buten-1-yloxy)penicillanate, as a yellow oil.

*Step C*

A solution of 6.3 g benzyl 6β-bromo-6α-(3-buten-1-yloxy)penicillanate (obtained in Step B) in 80 ml dichloromethane and 30 ml methanol is cooled to −20°C and ozonized oxygen is introduced until the starting material is consumed (as determined by t.l.c.). Then, 10 ml dimethyl sulfide is added, the resultant solution is brought to room temperature, and washed twice with 100 ml portions of water. The aqueous phases are combined and extracted with 50 ml dichloromethane. The dichloromethane extracts are combined with the original organic phase and dried over anhydrous magnesium sulfate. The dried solution is evaporated *in vacuo* at room temperature to leave benzyl 6α-(3-oxopropoxy)-6β-bromo-penicillanate as an oil.

*Step D*

A solution of 5.8 g benzyl 6α-(3-oxopropoxy)-6β-bromo-penicillanate (obtained in Step C) in 100 ml dry tetrahydrofuran is stirred under argon for 20 hours with 25 g zinc dust. The mixture is then diluted with 200 ml ethyl acetate and 10 ml water, and the solids filtered off and washed with ethyl acetate. The filtrate is washed with water, dried over anhydrous magnesium sulfate and evaporated. The residue is chromatographed on 80 g silica gel eluting rapidly with dichloromethane followed by 5% ethyl acetate in dichloromethane. The appropriate eluates are combined and evaporated to give a mixture of benzyl spiro-[3′-hydroxy oxacyclopentyl]-2′,6α-penicillanate and benzyl spiro[3′-hydroxy oxacyclopentyl]-2′,6β-penicillanate.

Separation is effected by preparative thin layer chromatography on 0.1 cm layer plates, using two elutions with 3.2 v/v hexane:ethyl acetate. The bands are removed and the products are eluted using ethyl acetate. After evaporation, the residues are recrystallized from ether-hexane to afford the two compounds as white prisms m.p. 105—106°C and as white prisms melting at 113—115°C. These may be represented by the formula

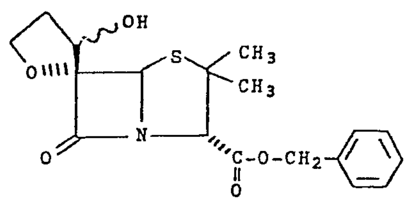

wherein the wavy line indicates the α- or β-configuration.

Example 14

A solution of 150 mg of benzhydryl 6β-(1-hydroxyethyl)penicillanate (prepared as in Example 3) in 25 ml ethanol is hydrogenolysed in the presence of a 10% palladium-on-carbon catalyst at 1.4 kg.cm$^{-2}$. After 24 hours, the solution is filtered to remove the catalyst and the solvent is evaporated. The resultant crystals, 6β-(1-hydroxyethyl) penicillanic acid, are treated with one equivalent of sodium bicarbonate in water. Freeze drying thus affords sodium 6β-(1-hydroxyethyl)penicillanate having the following spectral data:

NMR: ($CDCl_3$/DMSO on acid) 5.4 (d, J = 4.5$H_2$, 1H) 4.4 (s, 1H); 4.3 (m, 1H); 3.7 (dd, 1H); 1.6 and 1.5 (6H); 1.23 (d, 3H)

IR: (Na salt) 3400, 1700, 1600.

Example 15

A solution of 100 mg benzyl 7-(1-hydroxyethyl)desacetoxy-$\Delta^3$-cephalosporanate (prepared as in Example 6) in 20 ml ethanol is hydrogenated at 2.1 kg.cm.$^{-2}$ using a 10% palladium-on-carbon catalyst for 30 hours. The reaction mixture is then filtered to remove the catalyst and the solvent removed under vacuum to afford 7-(1-hydroxyethyl)desacetoxy-$\Delta^3$-cephalosporanic acid. This product is then treated with one equivalent of sodium bicarbonate and lyophilized to yield sodium 7-(1-hydroxyethyl)desacetoxy-$\Delta^3$-cephalosporanic acid having the following structural formula

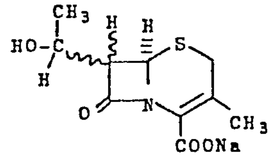

wherein the wavy line indicates the α- or β-configuration.

Example 16

A solution of 38 mg of benzhydryl 6β-(*o*-fluoro-α-hydroxybenzyl)penicillanate (prepared as in

Example 4) in 10 ml ethanol containing 2 mg N-methylglucamine is hydrogenated for 40 hours using a 30% palladium-on-carbon catalyst and atmospheric pressure. The reaction mixture is then filtered, the solvent removed under vacuum, and the resultant product washed with petroleum ether to remove any excess diphenylmethane. Thus produced is 6β-(*o*-fluoro-α-hydroxybenzyl)penicillanic acid having the following spectral data:

NMR: 7.2 (m, 4H); 5.5 (2H, exchangeable); 5.3 (d, 2J = 7, 2H); 5.2 (d, J = 4.5 $H_2$, 2H); 4.31 (s, 1H); 4.05 (dd, 1H); 1.7 and 1.4 (6H)
IR: 3400, 2700, 1770, 1730, 1600 $cm^{-1}$.

Example 17

A solution of 150 mg benzhydryl 6β-(1-hydroxyethyl)penicillanate (prepared as in Example 3) in 5 ml dry dimethylformamide is treated with 200 mg tosyl chloride followed by 200 mg triethylamine. After 12 hours, the reaction mixture is dissolved in ethyl acetate and then washed successively with water, 5% aqueous phosphoric acid, 1% aqueous sodium bicarbonate, and brine. After the solution is dried over anhydrous sodium sulfate, the solvents are removed under vacuum to afford, as a foam, benzhydryl 6β-(1-formyloxyethyl)penicillanate having the following spectral data:

MS: 439 ($M^+$)
NMR: 8.15 (s, 1H); 7.4 (10H); 7.0 (s, 1H); 5.65 (m, 1H); 5.4 (d, J = 5, 1H); 4.6 (s, 1H); 3.7 (dd, 1H); 1.6 (s, 3H); 1.3 (d, 3H); 1.25 (s, 3H).

Example 18

A solution of 411 mg benzhydryl 6β-(1-hydroxyethyl)penicillanate (prepared as in Example 3) in 10 ml dry acetonitrile is treated with 150 mg dry pyridine and 250 mg methanesulfonic anhydride. When the starting material is no longer present (as determined by thin layer chromatography), the reaction mixture is diluted with 50 ml ethyl acetate and washed first with 3 ml of 5% aqueous phosphoric acid, subsequently with 15 ml of 2% aqueous sodium bicarbonate and finally with brine. The resultant solution is dried over anhydrous sodium sulfate and the solvents removed under vacuum to afford, as a foamy solid, benzhydryl 6β-(1-methanesulfonyloxyethyl)penicillanate having the following spectral data:

NMR: 7.4 (10H); 7.0 (2, 1H); 5.4 (d, J = 6, 1H); 5.2 (m, 1H); 4.6 (s, 1H); 3.7 (dd, J = 4.5 and 6, 1H); 3.1 (s, 3H); 1.7 (s, 3H); 1.45 (d, 3H); 1.2 (s, 3H).

Example 19

A solution of 50 mg of benzhydryl 6β-(1-methanesulfonyloxyethyl)penicillanate (prepared as in Example 18) in 5 ml dry acetone is kept in the presence of 200 mg sodium iodide for 5 days. The acetone is then removed under vacuum and the residue is dissolved in 50 ml ethyl ester. The ether solution is washed first with water and then with aqueous sodium thiosulfate. After drying over anhydrous sodium sulfate, the solvent is removed under vacuum to afford, as a white solid, benzhydryl 6β-(1-iodoethyl)penicillanate, having the following spectral data:

NMR: 7.4 (10H); 7.0 (s, 1H); 5.4 (d, J = 5, 1H); 4.6 (s, 1H); 4.6—3.6 (2H); 2.0 (d, 3H); 1.6 (3H); 1.3 (s, 3H)
IR: 1770, 1740 $cm.^{-1}$.

Example 20

A solution of 0.6 g benzyl 6-(1-hydroxyethyl)-6-methoxy-penicillanate (prepared as in Example 8) in 30 ml ethanol and 10 ml water containing 0.2 g sodium bicarbonate is shaken for 20 hours with 1 g 10% palladium-on-charcoal catalyst at 4.2 kg. $cm.^{-2}$. The mixture is then filtered, the solid is washed with aqueous ethanol and the filtrate and washings are evaporated *in vacuo* to remove most of the ethanol. The residual aqueous solution is extracted once with ether and the extract discarded. The aqueous phase ia acidified to about pH 1.5 with phosphoric acid and then extracted 5 times with 50 ml portions of dichloromethane. The dichloromethane extracts are combined, dried over anhydrous magnesium sulfate and evaporated. The residue is recrystallized from ether-hexane to afford (6-(1-hydroxyethyl)-6-methoxypenicillanic acid as a white, crystalline solid melting at 201—204°C with decomposition. To an ethanol solution containing the free acid is added 1 ml of a 0.6 M dichloromethane solution of potassium 2-ethylhexanoate followed by 30 ml hexane. The precipitate is filtered off, washed with hexane and dried *in vacuo* at 50°C to afford, as a white powder, potassium 6-(1-hydroxyethyl)-6-methoxypenicillanate.

Example 21

Activated mossy zinc (500 mg) and zinc dust (100 mg) are added to a solution of 2,2-dimethyl-3α-cyano-6α-bromopenam (522 mg, 2 mmol) and *p*-(2-tetrahydropyranyloxy)benzaldehyde (750 mg,

3.75 mmol) (prepared according to E. Piers, W. deWaal and R. W. Britton, *J. Am. Chem. Soc., 93*, 5113 (1971)) in 1 ml of dry tetrahydrofuran. After vigorous stirring at 50° for 3 hrs., the mixture is allowed to cool and 1 ml of pH 5 phosphate buffer is added. After standing for 10 min., the solids are removed by filtration and washed thoroughly with ethyl acetate. The filtrate is extracted with ethyl acetate. The combined extracts are washed with phosphate buffer and saturated sodium chloride, dried with sodium sulfate and the solvents removed by evaporation. The crude material is purified by chromatography on 60 g of silica gel using 40% acetone/chloroform as eluant to afford 2,2-dimethyl-3α-cyanopenam as a by-product melting at 73—75°C and a mixture of 2,2-dimethyl-3α-cyano-6α-[*p*-(2-tetrahydropyranyloxy)-α-hydroxybenzyl]penam and 2,2-dimethyl-3α-cyano-6β-[*p*-(2-tetrahydropyranyloxy)-α-hydroxybenzyl]penam. Separation of the 6α- and 6β-compounds is effected by PLC (10% acetone/toluene; two developments) providing the individual 6α- and 6β-compounds having the following spectral data:

6α Compound
MS: 304 (m/e).
NMR: 8.0—6.8 (m, 4H); 5.35 (s, 1H and d, δ = 4, 1H); 5.15 (d, δ = 2, 1H); 5.08 (dd, δ = 2, 4, 1H); 4.70 (s, 1H); 3.70 (br m, 4H); 1.71, 1.56 (s, 3H).
IR: 3400, 1700 cm.$^{-1}$.

6β Compound:
MS: 388 ($M^+$)
NMR: 7.4—6.8 (m, 4H); 5.4 (br s, 2H); 5.10 (br m, 1H); 4.6 (s, 1H); 3.95 (dd, δ = 2, 4, 1H); 3.65 (br s, 1H); 1.71, 1.66 (s, 3H).
IR: 3400, 1770 cm.$^{-1}$.

## Example 22

2,2-Dimethyl-3α-cyano-6α-[*p*-(2-tetrahydropyranyloxy)-α-hydroxybenzyl]penam (prepared as in Example 21) (45 mg, 0.12 mmol) is dissolved in 3 ml of acetone and 0.5 ml of 10% phosphoric acid. After stirring 18 hours at room temperature, the solution is diluted with a 10 fold excess of ethyl acetate and washed with saturated sodium chloride. Purification of the crude phenol (42 mg) by PLC (35% acetone/toluene) provides 2,2-dimethyl-3α-cyano-6α-(*p*-hydroxy-α-hydroxybenzyl)penam having the following spectral data:

MS: 304 ($M^+$)
NMR: 7.4—6.7 (m, 4H); 5.34 (d, δ = 2, 1H); 5.13 (d, δ = 2, 1H); 4.97 (m, 1H); 4.71 (s, 1H); 3.63 (m, 1H); 1.68, 1.53 (s, 3H).
IR: 3400, 1770 cm.$^{-1}$.

The 6β-tetrahydropyranyl ether is deprotected in the same manner to provide 2,2-dimethyl-3α-cyano-6β-(*p*-hydroxy-α-hydroxybenzyl)penam having the following spectral data:

MS: 304 ($M^+$)
NMR: 7.27, 6.78 (d, δ = 10, 4H); 5.08 (d, δ = 4, 1H); 5.04 (d, δ = 9, 1H); 4.60 (s, 1H); 3.98 (dd, δ = 4, 9, 1H); 1.68, 1.64 (s, 3H).
IR: 3400, 1770 cm.$^{-1}$.

## Example 23

Activated mossy zinc (1.0 g) and zinc dust (1.2 g) is added to a solution of 2,2-dimethyl-3α-cyano-6α-bromopenam (1.0 g, 3.85 mmol) and *p*-diphenylmethoxycarbonyl-benzaldehyde (1.96 g, 6.2 mmol) in 6 ml of THF (distilled from calcium hydride). After stirring vigorously at room temperature for 18 hours, the mixture is diluted with ethyl acetate. Phosphate buffer (pH 5, 5 ml) is then added. After stirring for 10 min., the solids are removed by filtration and washed thoroughly with ethyl acetate. The layers of the filtrate are separated and the organic phase is washed with phosphate buffer and saturated sodium chloride. Drying with sodium sulfate and solvent removal provides a yellow foam.

Purification of the crude mixture on 60 g of silica gel 60 with 5% acetone/toluene as eluant affords 2,2-dimethyl-3α-cyano-6α-(p-diphenylmethoxycarbonyl-α-hydroxybenzyl)penam and 2,2-dimethyl-3α-cyano-6β-(*p*-diphenylmethoxy-carbonyl-α-hydroxybenzyl)penam having the following spectral data:

6α Compound:
MS: 498 ($M^+$)
NMR: 8.09 (d, δ = 8, 2H); 7.6—7.2 (m, 12H); 7.12 (s, 1H); 5.20 (d, δ = 2, 1H); 5.12 (m, 1H); 4.71 (s, 1H); 3.70 (dd, δ = 2, 1H); 1.70, 1.51 (s, 3H).
IR: 3400, 2200, 1770, 1720 cm.$^{-1}$.

6$\beta$ Compound:
MS: 498 ($M^+$)
NMR: 8.09 (d, $\delta=8$, 2H); 7.6—7.2 (m, 12H); 7.12 (s, 1H); 5.18 (d, $\delta=9$, 1H); 5.12 (d, $\delta=4$, 1H); 4.65 (s, 1H); 3.93 (dd, $\delta=4$, 9, 1H); 1.69, 1.66 (s, 3H).
IR: 3400, 2200, 1770, 1720 $cm.^{-1}$.

Example 24

2,2-Dimethyl-3$\alpha$-cyano-6$\alpha$-(*p*-diphenylmethoxycarbonyl-$\alpha$-hydroxybenzyl)penam (prepared as in Example 23) (0.26 mmol, 132 mg) is dissolved in 1.2 ml of anisol and cooled to 0°. Dry trifluoroacetic acid (3 ml.) is added and the solution is allowed to stand at 0° for 2 mins. The trifluoroacetic acid is removed under high vacuum at 0° and the anisol is distilled off at 30°. More anisol is added and distilled off. This is again repeated and the oily residue partitioned between 5 ml of cold water containing 22 mg of sodium bicarbonate and ether. The phases are separated and the aqueous phase is again washed with ether and lyophilized, affording sodium 2,2-dimethyl-3$\alpha$-cyano-6$\alpha$-(*p*-carboxy-$\alpha$-hydroxybenzyl)penam.

2,2-dimethyl-3$\alpha$-cyano-6$\beta$-(*p*-diphenylmethoxycarbonyl-$\alpha$-hydroxybenzyl)penam (62 mg, 0.12 mmol) is treated in the same manner to provide sodium 2,2-dimethyl-3$\alpha$-cyano-6$\beta$-(*p*-carboxy-$\alpha$-hydroxybenzyl)penam.

In a similar manner to that described in the foregoing Examples, the following compounds, having the spectral data shown, may be prepared:

1. 2,2-dimethyl-3$\alpha$-cyano-6-(*o*-benzyloxy-$\alpha$-hydroxybenzyl)penam:

6$\alpha$ Compound:
MS: 376 ($M^+$ —$H_2O$), 131, 91 (base)
NMR: 1.55 (s, 3H); 1.60 (s, 3H); 3.88 (m, 1H); 4.71, 4.72 (s, 1H); 5.15 (s, 2H); 5.32, 5.36 (d, $\delta=2$); 5.42 (br m, 1H); 7.12 (m, 4H); 7.44 (s, 5H).

6$\beta$ Compound:
MS: 376 ($M^+$ —$H_2O$); 163, 131, 91 (base)
NMR: 1.60 (s, 3H); 1.66 (s, 3H); 4.30 (dd, $\delta=5$, 7, 1H); 4.62 (s, 1H); 5.14 (s, 2H); 5.17 (d, $\delta=5$, 1H); 5.34 (br m, 1H); 7.18 (m, 4H); 7.40 (s, 5H).

2. 2,2-dimethyl-3$\alpha$-cyano-6-(*p*-phenylmethoxycarbonyl-$\alpha$-hydroxybenzyl)penam:

6$\alpha$ Compound:
MS: 422 ($M^+$), 175, 133, 91 (base)
NMR: 1.51 (s, 3H); 1.66 (s, 3H); 3.67 (m, 1H); 4.68 (s, 1H); 5.11 (d, $\delta=7$, 1H); 5.17 (d, $\delta=2$, 1H); 5.33 (s, 2H); 7.37 (br s, 7H); 8.05 (m, 2H).

6$\beta$ Compound:
MS: 422 ($M^+$), 175, 133, 91 (base)
NMR: 1.64 (s, 3H); 1.71 (s, 3H); 3.96 (dd, $\delta=5$, 10, 1H); 4.65 (s, 1H); 5.16 (d, $\delta=5$, 1H); 5.36 (br s, 3H); 7.39 (br s, 7H); 8.07 (m, 2H).

3. benzhydryl-6$\alpha$-(*p*-dimethylamino-$\alpha$-hydroxybenzyl)penicillanate.

MS: 516 ($M^+$), 232, 167 (base), 150.
NMR: 1.21 (s, 3H); 1.56 (s, 3H); 2.89 (s, 6H); 3.62 (dd, $\delta=2$, 6, 1H); 4.54 (s, 1H); 5.07 (d, $\delta=6$, 1H); 5.41 (d, $\delta=2$, 1H); 6.92 (m, 4H); 6.88 (s, 1H); 7.37 (s, 10H).

4. 7$\alpha$-(*p*-dimethylamino-$\alpha$-hydroxybenzyl)-4-cyano-3-cephem.

MS: 329 ($M^+$), 311, 158, 150 (base).
NMR: 2.09 (s, 3H); 2.14 (s, 3H); 2.96 (s, 6H); 3.22 (br s, 1H); 3.57 (m, 2H); 4.55 and 4.88 (d, $\delta$, = 3, 1H); 5.03 and 5.16 (d, $\delta=5$, 1H); 6.94 (m, 4H).

5. 2,2-dimethyl-3$\alpha$-cyano-6-(2′-thienylhydroxymethyl)penam.

The following spectral data, not assigned, was obtained for the isomers:

(i) MS: 294 ($M^+$), 192, 154, 113 (base).
NMR: 1.63 (s, 3H); 1.76 (s, 3); 4.06 (dd, $\delta=5$, 10H); 4.60 (s); 5.38 (m); 5.43 (d, $\delta=5$); 7.12 (m, 3H).

(ii) MS: 293 (M—1), 192 (base) 154, 113, 111.
NMR: 1.66 (s, 3H); 1.71—1.76 (s, 3H); 4.00 (dd, $\delta = 5$, 10H); 4.63 (s); 5.19 (d, $\delta = 5$); 5.38 (m, 1H); 7.14 (m, 3H).

## Formulation Examples

In the following formulation examples the designated active ingredients were as follows:

*Active ingredient A:* 2,2-dimethyl-3α-cyano-6α-(*p*-dimethylamino-α-hydroxybenzyl)penam.

*Active ingredient B:* 2,2-dimethyl-3α-cyano-6β-(*p*-diphenylmethoxycarbonyl-α-hydroxybenzyl)penam.

*Active ingredient C:* 2,2-dimethyl-3α-cyano-6β-(*p*-benzyloxy-α-hydroxybenzyl)penam.

### Formulation I

| Ointment | | mg/g |
|---|---|---|
| Active ingredient A, B, or C | | 0.001—0.1 |
| Mineral Oil | | 20.0 |
| White Petrolatum | To make | 1.00 g |

Procedure:
Melt and heat the white petrolatum to 60°—65°C. Disperse active ingredient in mineral oil and mill the suspension. Add the suspension to the melted white petrolatum with agitation. Start cooling and continue to agitate until the temperature reaches 25°C.

### Formulation 2

| Cream | | mg/g |
|---|---|---|
| Active ingredient A, B, or C | | 0.001—0.1 |
| Mineral Oil (viscosity 75 centistokes) | | 60.0 |
| White Petrolatum | | 150.0 |
| Cetostearyl Alcohol | | 72.0 |
| Polyethylene Glycol 1000 Monocetyl-Ether | | 22.5 |
| Propylene Glycol | | 100.0 |
| Sodium Phosphate, Monobasic | | 2.65 |
| Phosphoric Acid | | 0.02 |
| 4-chloro-m-cresol | | 1.0 |
| Purified Water | To make | 1,000.0 |

Procedure:
Melt together and heat to 70°C the white petrolatum, cetostearyl alcohol, mineral oil and polyethylene glycol 1000 monocetyl ether. Dissolve sodium phosphate, mono-basic, phosphoric acid and 4-chloro-m-cresol in water and heat to 70°C. Dissolve or suspend active ingredient in propylene glycol and add to the aqeuous phase and heat to 70°C. Add the aqueous phase to oily phase with agitation. Start cooling and continue to agitate until the temperature reaches 25°C.

Formulation 3

| Injectable suspension | mg/g |
|---|---|
| Active ingredient A (Sterile) | 250.0 |
| Benzyl Alcohol | 9.0 |
| Methylparaben | 1.8 |
| Propylparaben | 0.2 |
| Sodium Carboxymethylcellulose | 5.0 |
| Polyethylene-glycol (4000 grade) | 10.0 |
| Polyvinylpyrrolidone | 5.0 |
| Sodium Citrate | 15.0 |
| Disodium ethylenediamine tetraacetic acid (EDTA) | 0.1 |
| Water for Injection q.s. ad | 1.0 ml |

Method of Manufacture:

1. Dissolve parabens in a portion of the water for injection by heating it to 65—70°C.
2. Cool to 25—35°C. Charge and dissolve benzyl alcohol, sodium citrate, disodium EDTA, polyethylene glycol, polyvinylpyrrolidone and sodium carboxymethylcellulose.
3. Filter the solution and sterilize by autoclaving.
4. Make a slurry of the sterile active and pass it through a colloid mill.
5. Mix well the product from Step 4 with solution from Step No. 3 and pass it through the mill.
6. Bring the suspension to final volume/weight and fill into sterile containers.

Formulation 4

Injection (pervial)

Active ingredient A (Sterile Powder) 500 mg
For reconstitution add sterile water for injection

Formulation 5

Tablets

| Item No. | mg/tab. | mg/tab. |
|---|---|---|
| 1. Active ingredient A (micronized) or methylglucomine salt thereof | 250 | 500 |
| 2. Lactose USP | 97.5 | 195 |
| 3. Microcrystalline Cellulose | 25.0 | 50.0 |
| 4. Corn Starch (as 10% paste) | 25.0 | 50.0 |
| 5. Corn Starch, dry | 25.0 | 50.0 |
| 6. Microcrystalline Cellulose | 25.0 | 50.0 |
| 7. Magnesium Stearate | 2.50 | 5.00 |
| | 450 | 900 |

Method of Manufacture:

Mix item Nos. 1, 2 and 3 in a suitable mixer. Granulate the blend with the item No. 4. Pass the wet granulated mass through a suitable mill having a US sieve No. 6. Spread the damp granules on

trays and dry at 40°—50°C overnight. Pass the dried granules through a mill having a US standard sieve No. 16 to 20 and collect the milled granules in a suitable blender. Add item Nos. 5 and 6 and mix for 10—15 minutes. Add item No. 7 as a premix to the above mixture and blend further to 3—5 minutes. Compress the mixture on a suitable tablet machine into a tablet of desired shape, size and thickness.

## Claims

1. A process for the preparation of a $\beta$-lactam derivative having a substituted hydroxymethyl group at the carbon atom $\alpha$ to the lactam carbonyl group, characterised by reacting in an anhydrous aprotic medium, an $\alpha$-halo-$\beta$-lactam starting material with activated zinc or zinc amalgam in the presence of an appropriate aldehyde or ketone and hydrolysing the resulting zinc complex.

2. A process as claimed in claim 1, characterized in that hydrolysis is effected with an aqueous medium at a pH of 4 to 7.

3. A process as claimed in claim 1 or claim 2, characterized in that the $\alpha$-halo-$\beta$-lactam starting material is a 6-halopenicillanic acid derivative of the general formula XV

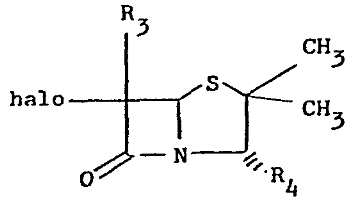

(XV)

or a 7-halocephalosporanic acid derivative of the general formula XVI

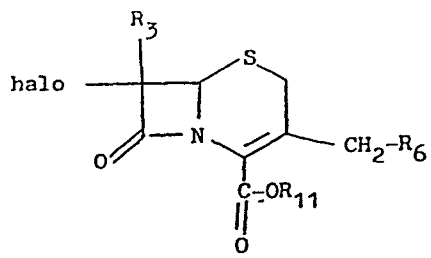

(XVI)

in which formulae, halo represents a halogen atom selected from chlorine, bromine or iodine, $R_3$ is hydrogen or lower alkoxy, $R_4$ is cyano or $COOR_{11}$ and $R_{11}$ is a readily removable ester forming moiety and $R_6$ is hydrogen, lower alkanoyloxy, lower alkoxy, lower alkylthio, pyridinium or a group

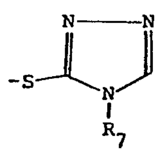 , 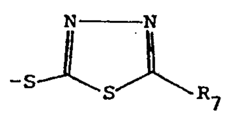 or 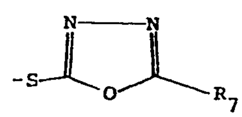

where $R_7$ is hydrogen, lower alkyl, phenyl, lower alkylphenyl, halophenyl, hydroxyphenyl, lower alkoxy-phenol or $R_7$ represents the group

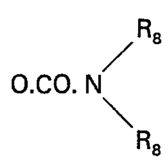

where each $R_8$ represents hydrogen or lower alkyl, the term 'lower' as applied to alkyl and alkoxy meaning that the indicated group has 1 to 6 carbon atoms and as applied to alkanoyl meaning that that group has 2 to 7 carbon atoms.

4. A process as claimed in any one of the preceding claims, characterised in that the zinc reagent employed is activated zinc.

5. A process as claimed in any one of the preceding claims, characterised in that an aromatic aldehyde or ketone is employed.

6. A process as claimed in any one of the preceding claims, characterised in that the aprotic medium is tetrahydrofuran or benzene.

7. A process as claimed in any one of the preceding claims, characterised in that the so-obtained $\beta$-lactam product having a substituted hydroxymethyl group at the carbon atom $\alpha$ to the lactam carbonyl group is further subjected, prior or subsequent to isolation and any separation into its stereochemical isomers, to one more of the steps of:

a) conversion on an appropriate function into a pharmaceutically acceptable salt,

b) removal of a protecting group,

c) conversion of the hydroxy group in the substituted hydroxymethyl moiety into an ether, ester or halo group, and

d) for a cephalosporin product bearing a 3-methylene substituent, isomerisation to the corresponding $\Delta^2$ and/or $\Delta^3$ cephem.

8. A $\beta$-lactam derivative of the general formula I

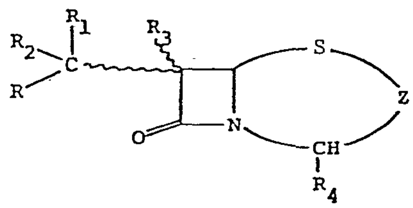

(I)

and the pharmaceutically acceptable salts thereof, wherein Z represents:

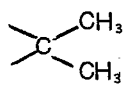
,
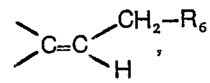
,
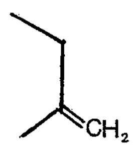
, or
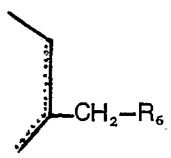

where the dotted line represents an optional double bond and $R_6$ represents hydrogen, lower alkanoyloxy, lower alkoxy, lower alkylthio, pyridinium or a group

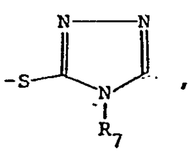
,
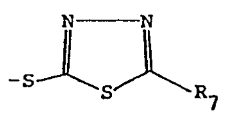
or
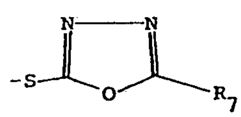

where $R_7$ is hydrogen, lower alkyl, phenyl, lower alkylphenyl, halophenyl, hydroxyphenyl, lower alkoxyphenyl or $R_7$ represents the group

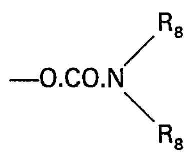

where each $R_8$ represents hydrogen or lower alkyl,

R represents OH, lower alkoxy, formyloxy, lower alkanoyloxy, lower alkylsulfonyloxy or halogen,

$R_1$ represents lower alkyl, lower alkenyl, lower alkynyl, lower cycloalkyl or a heterocyclic group containing an oxygen or sulfur heteroatom, all of which groupings may be substituted by a free or protected amino, hydroxy or carboxyl group, or more preferably $R_1$ represents substituted phenyl in which the phenyl group is substituted by one of more substituent groups selected from halo, nitro, lower alkyl, lower alkenyl, lower alkynyl, lower alkoxy, and free and protected-amino, -hydroxy and -carboxy groups.

$R_2$ represents hydrogen or is as defined for $R_1$,

$R_3$ represents hydrogen or lower alkyl or $R_3$ together with $R_1$ represents a divalent grouping A of the formula

$$—(CH_2)_m—(X)_p—(CH_2)_n—$$

in which grouping X is O or S, m and n independently are each 0 or an integer from 1 to 3, p is 0 or 1 with the proviso that m+n+p is 3, 4 or 5, and

$R_4$ represents cyano, a carboxylic acid group COOH or an ester thereof —$COOR_{11}$ where $R_{11}$ is a readily removable ester-forming moiety, with the proviso that when Z is

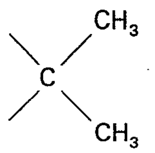
or
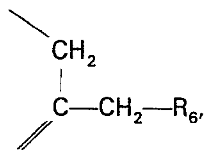

R is OH, $R_2$ and $R_3$ are hydrogen and $R_1$ is $CH_3$, then $R_4$ is —CN or a carboxylic acid ester grouping other than t-butyl or benzyl, the term 'lower' as applied to alkyl, alkenyl, alkynyl and alkoxy meaning that the indicated group has 1 to 6 carbon atoms, as applied to alkanoyl and alkanoyloxy meaning that the indicated group has 2 to 7 carbon atoms and as applied to cycloalkyl meaning that that group has 4 to 7 carbon atoms.

9. A compound as claimed in claim 8, characterised in that R is hydroxy, $R_2$ is hydrogen, $R_3$ is hydrogen or lower alkoxy containing 1 to 6 carbon atoms and Z, $R_1$ and $R_4$ are as defined in claim 8.

10. A compound as claimed in claim 9, characterised in that Z is

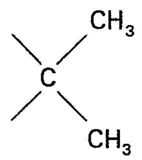

$R_3$ is hydrogen, $R_1$ is substituted phenyl as defined in claim 8 and $R_4$ is cyano.

11. A compound as claimed in claim 8, said compound being selected from:
benzyl spiro-[3'-hydroxy-oxacyclopentyl]-2',6α-penicillanate and,
benzyl spiro-[3'-hydroxy-oxacyclopentyl]-2',6β-penicillanate.

12. A compound as claimed in claim 9, said compound being selected from:
methyl .6β-(1-hydroxyethyl)penicillanate,
methyl-6α-(1-hydroxyethyl)penicillanate,
methyl 6β-(α-hydroxybenzyl)penicillanate,
methyl 6α-(α-hydroxybenzyl)penicillanate,
benzhydryl 6β-(1-hydroxyethyl)penicillanate,
benzhydryl-6α-(1-hydroxyethyl)penicillanate,
benzhydryl 6β-(o-fluoro-α-hydrobenzyl)penicillanate,
benzhydryl 6α-(o-fluoro-α-hydroxybenzyl)penicillanate,
benzhydryl 6β-(1-hydroxy-3-phenylprop-2-enyl)penicillanate,
benzhydryl 6α-(1-hydroxy-3-phenylprop-2-enyl)penicillanate,
benzyl-6-(1-hydroxyethyl)-6-methoxypenicillanate, and
potassium 6-(1-hydroxyethyl)-6-methoxypenicillanate.

13. A compound as claimed in claim 9, said compound being selected from:
benzylhydryl 7β-(1-hydroxyethyl)cephalosporanate,
benzhydryl 7α-(1-hydroxyethyl)cephalosoranate, and
sodium 7-(1-hydroxyethyl)desacetoxy-$\Delta^3$-cephalosporanic acid.

14. A compound as claimed in claim 10, said compound being selected from:
2,2-dimethyl-3α-cyano-6β-(1-hydroxyethyl)penam,
2,2-dimethyl-3α-cyano-6α-(1-hydroxyethyl)penam,
2,2-dimethyl-3α-cyano-6β-(α-hydroxybenzyl)penam,
2,2-dimethyl-3α-cyano-6α-(α-hydroxybenzyl)penam,
2,2-dimethyl-3α-cyano-6β-(p-dimethylamino-α-hydroxybenzyl)penam,
2,2-dimethyl-3α-cyano-6α-(p-dimethylamino-α-hydroxybenzyl)penam,
2,2-dimethyl-3α-cyano-6β-(p-benzyloxy-α-hydroxybenzyl)penam,
2,2-dimethyl-3α-cyano-6α-(p-benzyloxy-α-hydroxybenzyl)penam,
2,2-dimethyl-3α-cyano-6β-(p-diphenylmethoxycarbonyl-α-hydroxybenzyl)penam,
2,2-dimethyl-3α-cyano-6α-(p-diphenylmethoxycarbonyl-α-hydroxybenzyl)penam,
sodium 2,2-dimethyl-3α-cyano-6α-(p-carboxy-α-hydroxybenzyl)penam, and
sodium 2,2-dimethyl-3α-cyano-6β-(p-carboxy-α-hydroxybenzyl)penam.

15. A pharmaceutical composition comprising as active ingredient a compound as claimed in any one of claims 8 to 14 in association with a pharmaceutically acceptable carrier.

**Revendications**

1. Procédé pour la préparation d'un dérivé de β-lactame ayant un groupe hydroxyméthyle substitué à l'atome de carbone α du groupe carbonyl lactame, caractérisé en ce qu'on fait réagir, dans

un milieu aprotique anhydre, une matière première d'$\alpha$-halo-$\beta$-lactame avec du zinc activé ou un amalgame de zinc en présence d'un aldéhyde ou d'une cétone approprié et on hydrolyse le complexe de zinc résultant.

2. Procédé selon la revendication 1, caractérisé en ce que l'hydrolyse est effectuée avec un milieu aqueux àun pH de 4 à 7.

3. Procédé selon la revendication 1 ou la revendication 2, caractérisé en ce que la matière première d'$\alpha$-halo-$\beta$-lactame est un dérivé d'acide 6-halo-pénicillanique de formule générale XV

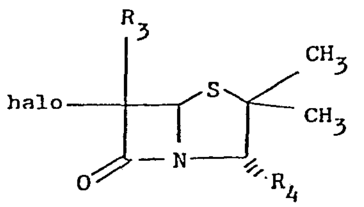

(XV)

ou un dérivé d'acide 7-halocéphalosporanique de formule générale XVI

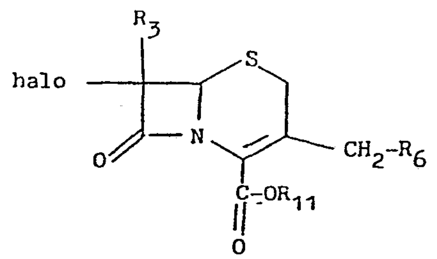

(XVI)

formules dans lesquelles halo représente un atome d'halogène choisi parmi le chlore, le brome ou l'iode, $R_3$ est de l'hydrogène ou un alcoxy inférieur, $R_4$ est cyano ou $COOR_{11}$ et $R_{11}$ est un fragment formant un ester qui est facilement amovible et $R_6$ est de l'hydrogène, un alcanoyloxy inférieur, un alcoxy inférieur, un alkylthio inférieur, un pyridinium ou un groupe

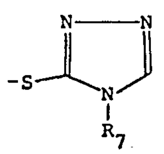 , 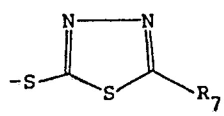 ou 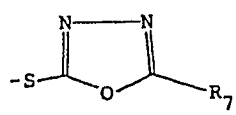

où $R_7$ est de l'hydrogène, un alcoyle inférieur, un phényle, un alkylphényle inférieur, un halophènyle, un hydroxyphényle, un alcoxyphényle inférieur ou $R_7$ représente le groupe

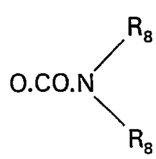

où chacun de $R_8$ représente de l'hydrogène ou un alcoyle inférieur, le terme "inférieur" appliqué à alcoyle ou alcoxy signifiant que le groupe indiqué a de 1 à 6 atomes de carbone et appliqué à alcanoyle signifiant que le groupe a 2 à 7 atomes de carbone.

4. Procédé selon l'une quelconque des revendications précédentes, caractérisé en ce que le zinc réactif employé est du zinc activé.

5. Procédé selon l'une quelconque des revendications précédentes, caractérisé en ce qu'on emploie un aldéhyde aromatique ou une cétone.

6. Procédé selon l'une quelconque des revendications précédentes, caractérisé en ce que le milieu aprotique est du tétrahydrofurane ou du benzène.

7. Procédé selon l'une quelconque des revendications précédentes, caractérisé en ce que le produit de $\beta$-lactame ainsi obtenu ayant un groupe hydroxyméthyle substitué à l'atome de carbone $\alpha$ du groupe carbonyl lactame est de plus soumis, avant ou après isolement et toute séparation en ses isomères stéréochimiques, à une ou plusieurs des étapes de:

a) conversion sur une fonction appropriée en un sel acceptable en pharmacie,

b) enlèvement d'un groupe protecteur,

c) conversion du groupe hydroxy dans le fragment hydroxyméthyle substitué en un groupe éther, ester ou halo, et

d) pour un produit de céphalosporine portant un substituant 3-méthylène, isomérisation en $\Delta^2$ et/ou $\Delta^3$ céphème correspondants.

8. Dérivé de $\beta$-lactame de formule générale I

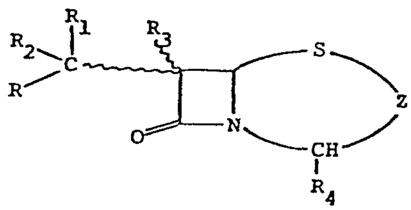

(I)

et ses sels acceptables en pharmacie, dans laquelle Z représente:

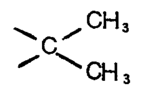 , 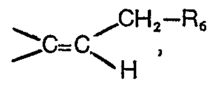 , 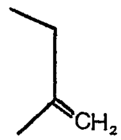 , ou 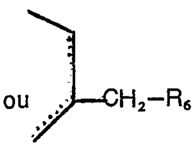

où la ligne en pointillés représente une double liaison éventuelle et $R_6$ représente de l'hydrogène, un alcanoyloxy inférieur, un alcoxy inférieur, un alkylthio inférieur, un pyridinium ou un groupe

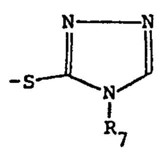 , 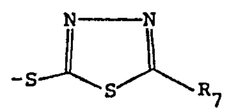 ou 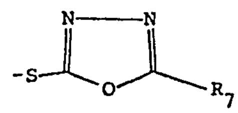

où $R_7$ est de l'hydrogène, un alcoyle inférieur, un phényle, un alkylphényle inférieur, un halophényle, un hydroxyphényle, un alcoxyphényle inférieur ou $R_7$ représente le groupe

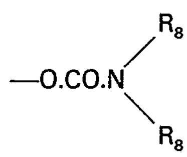

où chacun de $R_8$ représente de l'hydrogène ou un alcoyle inférieur,

R représente OH, un alcoxy inférieur, un formyloxy, un alcanoyloxy inférieur, un alkylsulfonyloxy inférieur ou un halogène,

$R_1$ représente un alcoyle inférieur, un alkényle inférieur, un alkynyle inférieur, un cycloalcoyle inférieur ou un groupe hétérocyclique contenant un hétéroatome d'oxygène ou de soufre, tous lesdits groupes pouvant être substitués par un groupe amino, hydroxy ou carboxyle libre ou protégé, ou de préférence $R_1$ représente un phényle substitué où le groupe phényle est substitué par un ou plusieurs groupes substituants choisis parmi les groupes halo, nitro, alcoyle inférieur, alkényle inférieur, alkynyle inférieur, alcoxy inférieur et groupes amino, hydroxy et carboxy libres et protégés.

$R_2$ représente de l'hydrogène ou est tel que défini pour $R_1$,

$R_3$ représente de l'hydrogène ou un alcoxy inférieur ou $R_3$ avec $R_1$ représente un groupage divalent A de formule

$$—CH_2)_m—(X)_p—(CH_2)_n—$$

groupage dans lequel X est O ou S, m et n sont indépendamment 0 ou un nombre entier de 1 à 3, p est 0 ou 1 à condition que l'on ait m+n+p= 3,4 ou 5, et représente un groupe cyano, acide carboxylique, COOH ou son ester —$COOR_{11}$ où $R_{11}$ est un fragment formant un ester facilement amovible, à condition que quand Z est

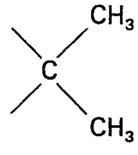 ou 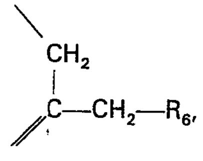

R soit OH, $R_2$ et $R_3$ soient de l'hydrogène et $R_1$ soit $CH_3$, alors $R_4$ est —CN ou un groupage d'ester d'acide carboxylique autre du t-butyle ou benzyle, le terme "inférieur" appliqué à alcoyle, alkényle,

alkynyle et alcoxy signifiant que le groupe indiqué a de 1 à 6 atomes de carbone, et appliqué à alcanoyle et alcanoyloxy signifiant que le groupe indiqué a de 2 à 7 atomes de carbone et appliqué à cycloalcoyle signifiant que le groupe a 4 à 7 atomes de carbone.

9. Composé selon la revendication 8, caractérisé en ce que R est hydroxy, $R_2$ est de l'hydrogène, $R_3$ est de l'hydrogène ou un alcoxy inférieur contenant de 1 à 6 atomes de carbone et Z, $R_1$ et $R_4$ sont tels que définis à la revendication 8.

10. Composé selon la revendication 9, caractérisé en ce que Z est

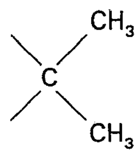

$R_3$ est de l'hydrogène, $R_1$ est un phényle substitué tel que défini à la revendication 8 et $R_4$ est cyano.

11. Composé selon la revendication 8, ledit composé étant choisi parmi:
spiro-(3'-hydroxy-oxaxyclopentyl)-2',6α-pénicillanate de benzyle et,
spiro-(3'-hydroxy-oxacyclopentyl)-2',6β-pénicillanate de benzyle.

12. Composé selon la revendication 9, ledit composé étant choisi parmi:
6β-(1-hydroxyéthyl)pénicillanate de méthyle,
6α-(1-hydroxyéthyl)pénicillanate de méthyle,
6β-(α-hydroxybenzyl)pénicillanate de méthyle,
6α-(α-hydroxybenzyl)pénicillanate de méthyle,
6β-(1-hydroxyéthyl)pénicillanate de benzhydryle,
6α-(1-hydroxyéthyl)pénicillanate de benzhydryle,
6β-(o-fluoro-α-hydroxybenzyl)pénicillanate de benzhydryle,
6β-(1-hydroxy-3-phénylprop-2-ényl)pénicillanate de benzhydryle,
6α-(o-fluoro-α-hydroxybenzyl)pénicillanate de benzhydryle,
6α-(1-hydroxy-3-phénylprop-2-ényl)pénicillanate de benzhydryle,
6-(1-hydroxyéthyl-6-méthoxypénicillanate de benzyle et 6-(1-hydroxyéthyl)-6-méthoxypénicillanate de potassium.

13. Composé selon la revendication 9, ledit composé étant choisi parmi:
7β-(1-hydroxyéthyl)céphalosporanate de benzhydryle,
7α-(1-hydroxyéthyl)céphalosporanate de benzhydryle et
acide 7-(1-hydroxyéthyl)désacétoxy-$\Delta^3$-céphalosporanique de sodium.

14. Composé selon la revendication 10, ledit composé étant choisi parmi:
2,2-diméthyl-3α-cyano-6β-(1-hydroxyéthyl)-péname,
2,2-diméthyl-3α-cyano-6α-(1-hydroxyéthyl)-péname,
2,2-diméthyl-3α-cyano-6β-(α-hydroxybenzyl)-péname,
2,2-diméthyl-3α-cyano-6α-(α-hydroxybenzyl)-péname,
2,2-diméthyl-3α-cyano-6β-(p-diméthylamino-α-hydroxybenzyl)péname.
2,2-diméthyl-3α-cyano-6α-(p-diméthylamino-α-hydroxybenzyl)péname,
2,2-diméthyl-3α-cyano-6β-(p-benzyloxy-α-hydroxybenzyl)péname,
2,2-diméthyl-3α-cyano-6α-(p-benzyloxy-α-hydroxybenzyl)péname,
2,2-diméthyl-3α-cyano-6β-(p-diphénylméthoxycarbonyl-α-hydroxybenzyl)péname,
2,2-diméthyl-3α-cyano-6α-(p-diphénylméthoxycarbonyl-α-hydroxybenzyl)péname,
sodium 2,2-diméthyl-3α-cyano-6α(p-carboxy-α-hydroxybenzyl)péname, et
sodium 2,2-diméthyl-3α-cyano-6β-(p-carboxy-α-hydroxybenzyl)péname.

15. Composition pharmaceutique contenant comme ingrédient actif un composé selon l'une quelconque des revendications 8 à 14 en association avec un véhicule acceptable en pharmacie.

## Patentansprüche

1. Verfahren zur Herstellung eines β-Lactam-Derivats mit einer substituierten Hydroxymethyl-Gruppe an dem Kohlenstoff-Atom in α-Stellung zu der Lactam-Carbonyl-Gruppe, dadurch gekennzeichnet, daß ein α-Halogeno-β-Lactam-Ausgangsmaterial mit aktiviertem Zink oder Zinkamalgam in einem wasserfreien aprotischen Medium in Gegenwart eines geeigneten Aldehyds oder Ketons umgesetzt wird und der entstehende Zink-Komplex hydrolysiert wird.

2. Verfahren nach Anspruch 1, dadurch gekennzeichnet, daß die Hydrolyse in einem wäßrigen Medium bei einem pH von 4—7 durchgeführt wird.

3. Verfahren nach Anspruch 1 oder 2, dadurch gekennzeichnet, daß das α-Halogeno-β-Lactam-Ausgangsmaterial ein 6-Halogeno-penicillansäure-Derivat der allgemeinen Formel XV

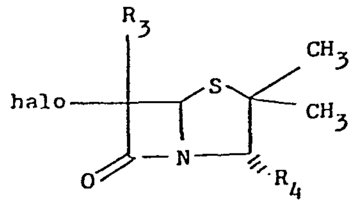

(XV)

oder ein 7-Halogenocephalosporansäure-Derivat der allgemeinen Formel XVI

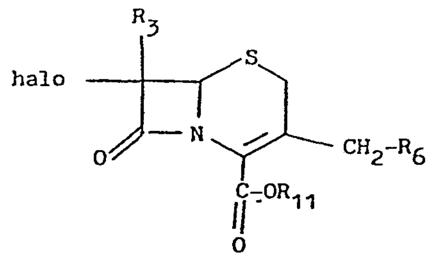

(XVI)

ist, wobei in diesen Formeln halo für ein Halogen-Atom, ausgewählt aus Chlor, Brom oder Iod steht, $R_3$ Wasserstoff oder niederes Alkoxy ist, $R_4$ Cyano oder $COOR_{11}$ und $R_{11}$ eine leicht entfernbare, esterbildende Struktureinheit ist und $R_6$ Wasserstoff, niederes Alkanoyloxy, niederes Alkoxy, niederes Alkylthio, Pyridinium oder eine Gruppe

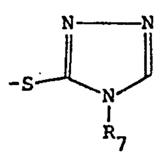 , 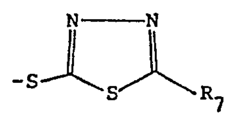 oder 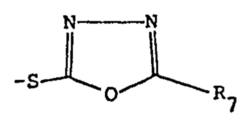

ist, worin $R_7$ Wasserstoff, niederes Alkyl, Phenyl, niederes Alkylphenyl, Halogenophenyl, Hydroxyphenyl, niederes Alkoxyphenyl ist oder für die Gruppe

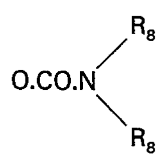

steht, worin jeweils $R_8$ für Wasserstoff oder niederes Alkyl steht, wobei der Begriff "nieder", wie er auf Alkyl oder Alkoxy angewandt wird, besagt, daß die bezeichnete Gruppe 1—6 Kohlenstoff-Atome besitzt, und wie er auf Alkanoyl angewandt wird, besagt, daß diese Gruppe 2—7 Kohlenstoff-Atome besitzt.

4. Verfahren nach einem der vorstehenden Ansprüche 1—3, dadurch gekennzeichnet, daß das verwendete Zink-Reagenz aktiviertes Zink ist.

5. Verfahren nach einem der vorstehenden Ansprüche 1—4, dadurch gekennzeichnet, daß ein aromatisches Aldehyd oder ein aromatisches Keton verwendet wird.

6. Verfahren nach einem der vorstehenden Ansprüche 1—5, dadurch gekennzeichnet, daß das aprotische Medium Tetrahydrofuran oder Benzol ist.

7. Verfahren nach einem der vorstehenden Ansprüche 1—6, dadurch gekennzeichnet, daß auf diese Weise erhaltene $\beta$-Lactam-Produkt mit einer substituierten Hydroxymethyl-Gruppe an dem Kohlenstoff-Atom in $\alpha$-Stellung zu der Lactam-Carbonyl-Gruppe weiterhin, vor oder nach seiner Isolierung und einer etwaigen Auftrennung in seine stereochemischen Isomeren, einem oder mehreren der Schritte

a) Umwandlung einer geeigneten Funktion in ein pharmazeutisch unbedenkliches Salz,

b) Entfernung einer Schutzgruppe,

c) Umwandlung der Hydroxy-Gruppe in der substituierten Hydroxymethyl-Struktureinheit in einen Ether, einen Ester, oder eine Halogenogruppe, und

d) im Falle eines Cephalosporin-Produktes, das einen 3-Methylen-Substituenten trägt, der Isomerisierung in das entsprechende $\Delta^2$ und/oder $\Delta^3$ Cephem
unterworfen wird.

8. $\beta$-Lactam-Derivat der allgemeinen Formel I

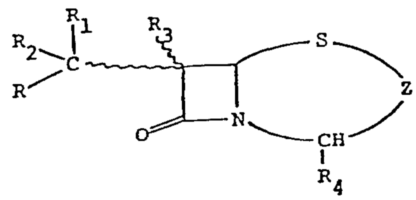

(I)

und dessen pharmazeutisch unbedenkliche Salze, wobei in dieser Formel Z für

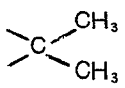 , 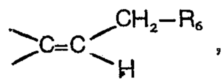 , 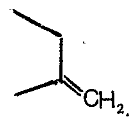 oder 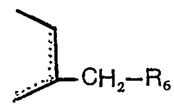

steht, worin die punktierte Linie eine gegebenenfalls vorhandene Doppelbindung bezeichnet und $R_6$ für Wasserstoff, niederes Alkanoyloxy, niederes Alkoxy, niederes Alkylthio, Pyridinium oder eine Gruppe

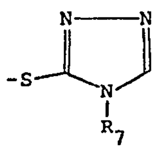 , 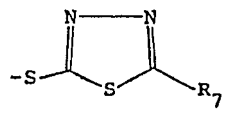 oder 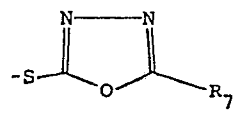

steht, worin $R_7$ Wasserstoff, niederes Alkyl, Phenyl, niederes Alkylphenyl, Halogenophenyl, Hydroxyphenyl, niederes Alkoxyphenyl ist oder die Gruppe

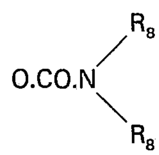

bezeichnet, worin jedes $R_8$ für Wasserstoff oder niederes Alkyl steht,

R für OH, niederes Alkoxy, Formyloxy, niederes Alkanoyloxy, niederes Alkylsulfonyloxy oder Halogen steht,

$R_1$ für niederes Alkyl, niederes Alkenyl, niederes Alkinyl, niederes Cycloalkyl oder eine heterocyclische, ein Sauerstoff- oder Schwefel- Heteroatom enthaltende Gruppe steht, wobei sämtliche dieser Gruppierungen durch eine freie oder geschützte Amino-, Hydroxy-, oder Carboxyl-Gruppe substituiert sein können, oder besonders bevorzugt $R_1$ für ein substituiertes Phenyl steht, in dem die Phenyl-Gruppe durch eine oder mehrere Substituentengruppen ausgewählt aus Halogen, Nitro, niederem Alkyl, niederem Alkonyl, niederem Alkinyl, niederem Alkoxy, sowie freien und geschützten Amino-, Hydroxy-, und Carboxy-Gruppen substituiert ist

$R_2$ für Wasserstoff steht oder die für $R_1$ gegebene Bedeutung hat,

$R_3$ für Wasserstoff oder niederes Alkoxy steht oder $R_3$ zusammen mit $R_1$ für eine zweiwertige Gruppierung A der Formel

$$—(CH_2)_m—(X)_p—(CH_2)_n—$$

steht, in der X O oder S ist, m and n unabhängig voneinander jeweils 0 oder eine ganze Zahl von 1—3 sind und p 0 oder 1 ist, mit der Maßgabe, daß m+n+p 3, 4 oder 5 ist, und

$R_4$ für Cyano, eine Carbonsäure-Gruppe COOH oder einen sich davon ableitenden Ester-$COOR_{11}$ steht, worin $R_{11}$ eine leicht entfernbare, esterbildende Struktureinheit ist, mit der Maßgabe, daß, wenn Z

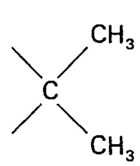 oder 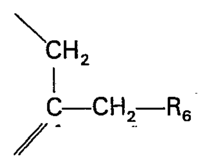

ist, R OH ist, $R_2$ und $R_3$ Wasserstoff sind und $R_1$ $CH_3$ ist, dann $R_4$ —CN oder eine Carbonsäureester - Gruppierung ist, die nicht t-Butyl oder Benzyl ist, wobei der Begriff "nieder", wie er auf Alkyl, Alkenyl, Alkinyl und Alkoxy angewendet wird, besagt, daß die bezeichnete Gruppe 1—6 Kohlenstoff-Atome besitzt, wie er auf Alkanoyl und Alkanoyloxy angewendet wird, besagt, daß die bezeichnete Gruppe 2—7 Kohlenstoff-Atome besitzt, und wie er auf Cycloalkyl angewandt wird, besagt, daß diese Gruppe 4—7 Kohlenstoff-Atome besitzt.

9. Verbindung nach Anspruch 8, dadurch gekennzeichnet, daß R Hydroxy, $R_2$ Wasserstoff und $R_3$ Wasserstoff oder niederes Alkoxy, das 1—6 Kohlenstoff-Atome enthält, ist, und Z, $R_1$ und $R_4$ die in Anspruch 8 festgelegten Bedeutungen haben.

10. Verbindung nach Anspruch 9, dadurch gekennzeichnet, daß Z

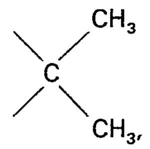

$R_3$ Wasserstoff, $R_1$ ein substituiertes Phenyl wie in Anspruch 8 definiert und $R_4$ Cyano ist.

11. Verbindung nach Anspruch 8, dadurch gekennzeichnet, daß sie ausgewählt ist aus Benzylspiro-[3'-hydroxy-oxacyclopentyl]-2', 6α-penicillanat und Benzylspiro-[3'-hydroxy-oxacyclopentyl]-2', 6β-penicillanat.

12. Verbindung nach Anspruch 9, dadurch gekennzeichnet, daß sie ausgewählt ist aus:
Methyl-6β-(1-hydroxyethyl)penicillanat,
Methyl-6α-(1-hydroxyethyl)penicillanat,
Methyl-6β-(α-hydroxybenzyl)penicillanat,
Methyl-6α-(α-hydroxybenzyl)penicillanat,
Benzhydryl-6β-(1-hydroxyethyl)penicillanat,
Benzhydryl-6α-(1-hydroxyethyl)penicillanat,
Benzhydryl-6β-(o-fluoro-α-hydroxybenzyl)penicillanat,
Benzhydryl-6α-(o-fluoro-α-hydroxybenzyl)penicillanat,
Benzhydryl-6β-(1-hydroxy-3-phenylprop-2-enyl)penicillanat,
Benzhydryl-6α-(1-hydroxy-3-phenylprop-2-enyl)penicillanat,
Benzyl-6-(1-hydroxyethyl)-6-methoxypenicillanat und Kalium-6-(1-hydroxyethyl)-6-methoxypenicillanat.

13. Verbindung nach Anspruch 9, dadurch gekennzeichnet, daß sie ausgewählt ist aus:
Benzylhydryl-7β-(1-hydroxyethyl)cephalosporanat,
Benzhydryl-7α-(1-hydroxyethyl)cephalosporanat, und
Natrium-7-(1-hydroxyethyl)desacetoxy-$\Delta^3$-cephalosporansäure.

14. Verbindung nach Anspruch 10, dadurch gekennzeichnet, daß sie ausgewählt ist aus:
2,2-Dimethyl-3α-cyano-6β-(1-hydroxyethyl)-penam,
2,2-Dimethyl-3α-cyano-6α-(1-hydroxyethyl)penam,
2,2-Dimethyl-3α-cyano-6β-(α-hydroxybenzyl)penam,
2,2-Dimethyl-3α-cyano-6α-(α-hydroxybenzyl)penam,
2,2-Dimethyl-3α-cyano-6β-(p-dimethylamino-α-hydroxybenzyl)penam,
2,2-Dimethyl-3α-cyano-6α-(p-dimethylamino-α-hydroxybenzyl)-penam,
2,2-Dimethyl-3α-cyano-6β-(p-benzyloxy-α-hydroxybenzyl)penam,
2,2-Dimethyl-3α-cyano-6α-(p-benzyloxy-α-hydroxybenzyl)penam,
2,2-Dimethyl-3α-cyano-6β-(p-diphenylmethoxycarbonyl-α-hydroxybenzyl)penam,
2,2-Dimethyl-3α-cyano-6α-(p-diphenylmethoxycarbonyl-α-hydroxybenzyl)penam,
Natrium-2,2-dimethyl-3α-cyano-6α-(p-carboxy-α-hydroxybenzyl)-penam und
Natrium-2,2-dimethyl-3α-cyano-6β-(p-carboxy-α-hydroxybenzyl)-penam.

15. Pharmazeutische Zusammensetzung, enthaltend als Wirkstoff eine Verbindung nach Anspruch 8—14 in Kombination mit einem pharmazeutisch unbedenklichen Träger.